(19) **Europäisches Patentamt / European Patent Office / Office européen des brevets**

(11) **EP 4 424 719 A1**

(12) # EUROPEAN PATENT APPLICATION
published in accordance with Art. 153(4) EPC

(43) Date of publication:
04.09.2024 Bulletin 2024/36

(21) Application number: 22886947.5

(22) Date of filing: 24.10.2022

(51) International Patent Classification (IPC):
C08F 2/48 (2006.01)     A61K 8/84 (2006.01)
A61Q 3/02 (2006.01)     B41M 5/00 (2006.01)
C08F 220/00 (2006.01)     C09D 4/00 (2006.01)
C09D 7/63 (2018.01)     C09D 11/101 (2014.01)
C09D 11/30 (2014.01)     C09D 11/38 (2014.01)
C09J 4/00 (2006.01)     C09J 7/38 (2018.01)
C09K 3/10 (2006.01)

(52) Cooperative Patent Classification (CPC):
A61K 8/84; A61Q 3/02; B41M 5/00; C08F 2/48;
C08F 220/00; C09D 4/00; C09D 7/63;
C09D 11/101; C09D 11/30; C09D 11/38;
C09J 4/00; C09J 7/38; C09K 3/10

(86) International application number:
PCT/JP2022/039517

(87) International publication number:
WO 2023/074620 (04.05.2023 Gazette 2023/18)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 25.10.2021 JP 2021174181
31.03.2022 JP 2022061018

(71) Applicant: KJ Chemicals Corporation
Tokyo 103-0023 (JP)

(72) Inventors:
• HIRATA Meiri
Tokyo 103-0023 (JP)
• KOBAYASHI Daisuke
Yatsushiro-shi Kumamoto 866-0081 (JP)
• TAKEDA Yoshimi
Yatsushiro-shi Kumamoto 866-0081 (JP)

(74) Representative: ABG Intellectual Property Law,
S.L.
Avenida de Burgos, 16D
Edificio Euromor
28036 Madrid (ES)

(54) **ACTIVE-ENERGY-RAY-CURABLE COMPOSITION**

(57) [Problem] To provide: a highly transparent active-energy-ray-curable composition that has few residual unreacted substances or low-molecular decomposition products, that does not cause odor or bleed-out, that makes it possible to acquire a cured product having high safety, durability, and transparency, and that can be cured completely even by long-wavelength light having a wavelength of 350 nm or greater; and a molded article which has various applications and in which the aforementioned composition is used.

[Solution] An active-energy-ray-curable composition that includes a polymerizable photoinitiator (A) having one or more benzophenone groups and one or more ethylenically unsaturated groups per molecule, and a polymerizable compound (B) (excluding A) having one or more ethylenically unsaturated groups per molecule, in which a content of components having a molecular weight of less than 1000 in a cured product of the aforementioned composition is less than 10%.

## Description

TECHNICAL FIELD

The present invention relates to an active energy ray curable composition, active energy ray curable pressure-sensitive adhesive agents, adhesive agents, sealing materials, inks, paints, coating agents, dental materials and cosmetic materials containing the composition, and cured products obtained by curing the composition, these agents or materials.

BACKGROUND ART

A photo-curing reaction using active energy rays such as ultraviolet rays (UV) is generally a reaction in which: a composition, in which a photopolymerization initiator is added, is irradiated with light to generate radicals (a radical polymerization system) or ions (a cationic polymerization system or an anionic polymerization system); a source material having unsaturated groups, epoxy groups, or the like is polymerized; and the liquid composition is solidified (cured) in a short time; and is used in a wide range of fields such as in paints and coating materials, pressure-sensitive adhesive materials and adhesives, elastomer-based materials, inkjet inks, materials for sealing and caulking materials, dental hygiene materials, and optical materials. In particular, since the photocurable resin can be cured in any place and in any shape, the photocuring reaction is widely used in the field of nail cosmetics such as gel nails and the field of 3D printers using three-dimensional modeling material.

Among such compositions, a photocurable resin composition using a radical photopolymerization initiator is widely used because curability is high and a wide range of physical properties can be achieved by combination with general-purpose monofunctional or polyfunctional (meth)acrylic monomers, oligomers or polymers in which (meth)acrylate groups are introduced, or the like. In recent years, as UV curable inks, paints and the like have become widespread, UV curing apparatuses have become larger, and there have been increasing demands for enhanced safety of light sources, such as preventing emission of ultraviolet rays called UV-B and UV-C having wavelengths of 315 nm or less considered to adversely affect the human body, and limiting the use of high-pressure mercury lamps according to the Minamata Treaty. However, the main light emitted from these light sources is 365 nm (black light, UV-LED), 385 nm (UV-LED) and 405 nm (LED lamp), and even if the stability of the light can be ensured, the absorption wavelengths of general-purpose photopolymerization initiators are mostly 350 nm or less, absorption of 375 nm or more is hardly observed, incomplete hardening or a long hardening time is required, and there is a new problem that the physical properties of a hardened product by non-uniform hardening are not satisfactory.

Examples of the radical photopolymerization initiator include an intramolecular cleavage type, and a hydrogen abstraction type, both of which generate a radical active species by light irradiation. A photopolymerization initiator of the intramolecular cleavage type has high efficiency of photopolymerization initiation, but it has low stability to heat and hence there has a problem with preservation stability of the initiator and a resin composition in which it is blended. Further, unreacted initiator and residues after reaction (intramolecular cleavage) remain as low molecular compounds in the cured product, and they bleed-out from the cured product over time; therefore, these residual low molecular compounds cause reduction in physical properties of the cured product, reduction in durability, generation of offensive odors, etc., and have been seen as a problem particularly with safety.

A photopolymerization initiator of the hydrogen abstraction type has a diaryl ketone structure like benzophenone, which extracts hydrogen from a hydrogen donor and generates a radical active species; therefore, the problem of residues after reaction can be remedied, and in recent years, it has been increasingly receiving attention. However, in general, the hydrogen abstraction type photopolymerization initiator has high stability to heat but low efficiency of photopolymerization initiation, and needs to be used in combination with a hydrogen donor such as amine, a photosensitizer, or the like as an additive. Since many of these additives are low molecular weight compounds, if these remains in the cured product, they may cause problems such as deterioration of the physical properties of the cured product due to bleed-out, deterioration of durability, odor and staining, and at the same time, problems of discoloration over time may occur.

In order to improve the efficiency of generation of a radical active species, which is an issue of the benzophenones-based photopolymerization initiator of the hydrogen abstraction type, Patent Literature 1 discloses the synthesis of a benzophenone derivative having a large number of peracid ester structures in the molecule, as a high-sensitivity photopolymerization initiator. However, although sensitivity has been enhanced successfully by the introduction of peracid ester structures, the peracid ester structure itself is known to be likely to decompose because of light or heat, and there has been a problem that low molecular compounds are generated as residual substances after polymerization reaction by light irradiation.

Patent Literature 2 discloses a high molecular photopolymerization initiator having a benzophenone group as a photoactive portion and an amine functional group or a tertiary amino group, which acts as a co-initiator. According to Patent Literature 2, since the high molecular photopolymerization initiator contains the amino group, inhibition by oxygen is reduced, and the speed of curing can be improved. However, both the amine and the amino group are generally recognized as functional groups having amine odors, and further it is known that these functional groups are very likely to color because of light irradiation. Furthermore, the photopolymerization initiator of Patent Literature 2 has a high molecular weight, and therefore exhibits low mobility; hence, generally has a problem that both the efficiency of production of radicals and the reactivity of photopolymerization (the speed of curing) are reduced.

On the other hand, in an active energy ray curing system, polymerizable compounds are used as an essential component. The polymerizable compounds remain in the monofunctional polymerizable compounds having one polymerizable functional group per one molecule and polyfunctional polymerizable compounds having two or more polymerizable functional groups per one molecule are contained, but in either case, most of the compounds have low molecular weights, and unless they are completely reacted and fixed in a cured product, are contained as low molecular weight components in cured products and cause deterioration in durability of the cured products due to odor and bleed-out in the same manner as the decomposition products of the photopolymerization initiator.

The present applicant has already disclosed a highly safe photopolymerization initiator having benzophenone structure and ethylenically unsaturated bonds (Patent Literature 3). It is shown that such a photopolymerization initiator does not produce a low molecular weight decomposition product as a by-product by photoreaction, can enter into a cured product via a strong chemical bond, and by using the photopolymerization initiator, the odor problem of the cured product caused by decomposition of the photopolymerization initiator and the problem of bleeding out of the decomposition product of the initiator over time can be solved. However, when the photopolymerization initiator described in this document is used, the residue of the photopolymerization initiator after curing is certainly incorporated into the cured product as a structural unit, but the total amount of low molecular weight components present in the cured product is not described, and in addition, as described in Example ([0105]) of this document, the curability with respect to long-wavelength rays, particularly 405 nm LED rays, cannot yet be said to be sufficient.

Therefore, there has not yet been found an active energy ray curable composition which has high curability to high-safety long-wavelength LED light and a low content of low molecular weight components in a cured product and can provide a high-safety cured product.

CITATION LIST

Patent Literature

Patent Literature 1: JP 2000-159827 A
Patent Literature 2: JP 22013-500303 A
Patent Literature 3: WO2021/117880

SUMMARY OF THE INVENTION

Technical Problem

The present invention is to provide an active energy ray curable composition which has high curability to active energy rays including long-wavelength LED rays, contains few unreacted materials and low-molecular decomposition products, does not cause odor and bleed-out problems, is excellent in compatibility, and can be completely cured even with long-wavelength rays of $\geq$ 350 nm wavelengths, and to provide active energy ray curable adhesive agents, pressure-sensitive adhesive agents, sealing materials, inks, paints, coating agents, dental materials and cosmetic materials containing the composition, and to provide cured products obtained by curing the same.

Solution to Problem

The present inventors conducted extensive studies and have found an active energy ray curable composition that contains a polymerizable photoinitiator (A) having one or more benzophenone groups and one or more ethylenically unsaturated groups per one molecule and a polymerizable compounds (B) (excluding A) having one or more ethylenically unsaturated group per one molecule. The polymerizable photoinitiator (A) has high photoinitiation efficiency, no by-products of low-molecular decomposition products, and is highly safe, and the polymerizable compound (B) is highly curable, furthermore, the polymerizable photoinitiator (A) and the polymerizable compound (B) have good compatibility, and a highly transparent curable composition and cured product applicable to the optical field can be obtained. In addition, the present inventors have found that the composition can be completely cured with light in a wide range of ultraviolet wavelengths including long wavelengths close to the visible light region, and the content of components with a molecular weight of less than 1000 in the cured product can be controlled to less than 10%, and a cured product having low odor, high safety, excellent water resistance, durability, etc., thus, have arrived at the present invention.

That is, the present invention provides the following:

(1) an active energy ray curable composition comprising a polymerizable photoinitiator (A) having one or more benzophenone groups and one or more ethylenically unsaturated groups per one molecule, and a polymerizable compound (B) (excluding A) having one or more ethylenically unsaturated group per one molecule, wherein a content of components having a molecular weight of less than 1000 in a cured product of the active energy ray curable composition is less than 10%,

(2) the active energy ray curable composition according to (1) above, wherein the polymerizable photoinitiator (A) and/or the polymerizable compound (B) have one or more covalent bonds of heteroatoms and hydrogen atoms per one molecule,

(3) the active energy ray curable composition according to (1) or (2) above, wherein the polymerizable photoinitiator (A) and/or the polymerizable compound (B) have one or more ethylenically unsaturated groups which may be selected from (meth)acrylamide group, (meth)acrylate group, vinyl group, vinyl ether group, alkyl vinyl ether group, allyl group, (meth)allyl ether group, styryl group, and maleimide group,

(4) the active energy ray curable composition according to any one of (1) to (3) above, wherein the polymerizable photoinitiator (A) and/or the polymerizable compound (B) form covalent bonds with hydrogen atoms using one or more heteroatoms which may be selected from oxygen atom, sulfur atom, nitrogen atom, phosphorus atom, boron atom, and silicon atom,

(5) the active energy ray curable composition according to any one of (1) to (4) above, wherein the polymerizable photoinitiator (A) has one or more (meth)acrylamide groups as the ethylenically unsaturated groups and one or more urethane bonds and/or urea bonds as the covalent bonds between heteroatoms and hydrogen atoms,

(6) an active energy ray curable ink composition containing the active energy ray curable composition according to any one of (1) to (5) above,

(7) an active energy ray curable ink jet ink composition containing the active energy ray curable composition according to any one of (1) to (5) above,

(8) an active energy ray curable ink composition for two dimensional or three-dimensional modeling containing the active energy ray curable composition according to any one of (1) to (5) above,

(9) an active energy ray curable nail cosmetic composition containing the active energy ray curable composition according to any one of (1) to (5) above,

(10) an active energy ray curable pressure-sensitive adhesive composition containing the active energy ray curable composition according to any one of (1) to (5) above,

(11) an active energy ray curable adhesive composition containing the active energy ray curable composition according to any one of (1) to (5) above,

(12) an active energy ray curable sealing material composition containing the active energy ray curable composition according to any one of (1) to (5) above,

(13) an active energy ray curable coating agent composition containing the active energy ray curable composition according to any one of (1) to (5) above,

(14) an active energy ray curable self-repairing coating material containing the active energy ray curable composition according to any one of (1) to (5) above, and

(15) an active energy ray curable dental composition containing the active energy ray curable composition according to any one of (1) to (5) above.

Advantageous Effects of Invention

According to the present invention, an active energy ray curable composition containing a polymerizable photoinitiator (A) having one or more benzophenone groups and one or more ethylenically unsaturated groups per one molecule and a polymerizable compounds (B) having one or more ethylenically unsaturated groups per one molecule is excellent in photopolymerization initiating property and photocuring property, can be completely cured by highly safe long-wavelength light, and a content of low molecular weight components with a molecular weight of less than 1000 in a cured product thereof is less than 10%, and a cured product with high durability and high safety without causing odor or bleed-out is possible to obtain. The active energy ray curable composition can be suitably used for various applications, such as ink, inkjet ink and photocurable ink for three- dimensional modeling, ink for two dimensional or three-dimensional modeling, nail cosmetics, pressure-sensitive adhesives, adhesives, sealing materials, coating agents, self-repairing paints, coating agents for vehicles, coating agents for building materials, and decorative films. The polymerizable photoinitiator (A) and the polymerizable compound (B) have good compatibility, the active energy ray curable composition of the present invention exhibits good transparency, and can be suitably used in various applications in the optical field such as an optical pressure-sensitive adhesive sheet, an optical adhesive, and an optical sealing material.

Description of Embodiments

Hereinbelow, embodiments of the present invention are described in detail.

An active energy ray curable composition of the present embodiment includes a polymerizable photoinitiator (A) having one or more benzophenone groups and one or more ethylenically unsaturated groups and a polymerizable compound (B) (excluding A) having one or more ethylenically unsaturated groups, and a content of components having a molecular weight of less than 1,000 in a cured product thereof is less than 10%. Both the polymerizable photoinitiator (A) and the polymerizable compound (B) contained in the curable composition are polymerizable compounds, and as photopolymerization proceeds by radicals generated by irradiation with active energy rays, both A and B are fixed as constituent units in the cured product via covalent bonds. In addition, both the polymerizable photoinitiator (A) and the polymerizable compound (B) are not degradable by active energy rays, and there is no by-product of low molecular weight components such as decomposition products accompanying photopolymerization, the resulting cured product has a low content of low molecular weight components and is characterized by excellent various performances of the cured product. In the cured product obtained, the content of components having a molecular weight of less than 1,000 is less than 10%, the content of components having a molecular weight of less than 1,000 is preferably less than 5%, the content of components having a molecular weight of less than 1,000 is more preferably less than 2%, and the content of components having a molecular weight of less than 500 is particularly preferably less than 2%.

The polymerizable photoinitiator (A) has one or more benzophenone groups and one or more ethylenically unsaturated groups per molecule. From the viewpoint of improving the polymerization initiating property and curability with respect to long-wavelength light and from the viewpoint that the polymerizable photoinitiator (A) can surely enter the cured product through a covalent bond by the polymerization reaction, the number of benzophenone groups per molecule is preferably 2 or more, and the number of ethylenically unsaturated groups is also preferably 2 or more. From the viewpoint that a curable composition containing a high content of polymerizable photoinitiator (A) requires a sufficient pot-life, it is preferred that the number of benzophenone groups per molecule does not exceed 50 and the number of ethylenically unsaturated groups does not exceed 12. From these viewpoints, it is more preferable that the number of the benzophenone groups per molecule is 2 to 30 and the number of the ethylenically unsaturated groups per molecule is 2 to 8, and it is particularly preferable that the number of the benzophenone groups is 4 to 12 and the number of the ethylenically unsaturated groups is 2 to 6. A ratio of the number of benzophenone groups and ethylene unsaturated groups is preferably 1/10 to 10/1, more preferably 1/8 to 8/1, and particularly preferably 1/5 to 5/1. When this number ratio is less than 0.1 (1/10), the polymerizable photoinitiator (A) is not fixed as a structural unit in the cured product through a covalent bond even after completion of the photopolymerization (curing) reaction, and as a result, the residue (unreacted portion) of A is present in a free state in the cured product, and especially when the molecular weight of the polymerizable photoinitiator (A) is less than 1000, A, as a low molecular weight component, tends to cause problems such as odor of the cured product, problems with bleed-out over time, problems with coloring, and problems with decreased durability. On the other hand, when this number ratio exceeds 10.0 (10/1), although depending on the content of the polymerizable photoinitiator (A) in the active energy ray curable composition, the polymerization rate of the curable composition significantly increases, and the temperature rapidly increases due to the heat of polymerization, and there are disadvantages in that it is difficult to use as a pressure-sensitive adhesive for plastic substrates, an adhesive, a sealing material for optical members, or as a nail cosmetic such as a gel nail.

The ethylenically unsaturated group of the polymerizable photoinitiator (A) is one or more groups selected from the group consisting of (meth)acrylamide group, (meth)acrylate group, vinyl group, vinyl ether group, methyl vinyl ether group, allyl group, (meth)allyl ether group, styryl group and maleimide group. When polymerizable photoinitiator (A) has two or more ethylenically unsaturated groups, they may be the same or different. Furthermore, the ethylenically unsaturated group preferably has at least one of (meth)acrylamide group, (meth)acrylate group, vinyl group and allyl group, more preferably at least one of (meth)acrylamide group and (meth)acrylate group, and particularly preferably at least one of (meth)acrylamide group. Since carbonyl in (meth)acrylamide group or (meth)acrylate group absorbs long-wavelength light, the absorption wavelengths in polymerizable photoinitiator (A) are shifted to the long-wavelength side due to the influence thereof, the photo-curing reaction becomes more safe because high-energy and dangerous short-wavelength light does not needs to be used, and further, since the polymerizability of (meth)acrylamide group is high, residues of polymerizable photoinitiator (A) and decomposition products when they are decomposed are more reliably fixed in the cured product through covalent bonds.

The benzophenone group of the polymerizable photoinitiator (A) has a diaryl ketone structure and is activated by irradiation with active energy rays, extracts a hydrogen atom from a hydrogen-donating functional group or compound, and the functional group or compound from which the hydrogen atom has been extracted becomes a free radical, and the free radical has high activity and becomes an actual photopolymerization initiating radical. Such hydrogen-donating functional groups or compounds are called coinitiators or polymerization synergists and are characterized by having a hydrogen atom covalently bonded to a heteroatom or a hydrogen atom covalently bonded to a carbon atom adjacent to a heteroatom (alpha or beta position). Since the bond energy of a covalent bond between a heteroatom and a hydrogen atom is lower than that of a covalent bond between a carbon atom and a hydrogen atom, a highly active free radical can be more easily generated by having a covalent bond between a heteroatom and a hydrogen atom in the active energy ray curable composition, which is preferable.

In the present embodiment, the coinitiator may be the polymerizable photoinitiator (A) itself, may be the polymerizable compound (B), or may further use a compound other than A and B. When the polymerizable photoinitiator (A) has a covalent bond between a heteroatom and a hydrogen atom in its molecule, it can play two roles as an initiator and a coinitiator. The polymerizable photoinitiator (A) preferably has one or more covalent bonds of a heteroatom and a hydrogen atom per molecule, and more preferably has two or more covalent bonds. The polymerizable photoinitiator (A) can extract hydrogen both within and between molecules, and in order to facilitate intramolecular hydrogen abstraction, the polymerizable photoinitiator (A) preferably has three or more linking atoms, more preferably five or more linking atoms, and most preferably seven or more linking atoms between the heteroatoms and the oxygen atom of the ketone bond in the benzophenone group. When there are three or more linking atoms between the heteroatoms and the oxygen atom of the ketone bond in the benzophenone group, the oxygen atom is more likely to approach the hydrogen atoms bonded to the heteroatoms, and the benzophenone group is more likely to abstract the hydrogen atoms from the heteroatoms. In view of the accessible distances between the oxygen atom of the ketone bond in benzophenone group and the hydrogen atoms described above, it is preferable to have 15 or less linking atoms between the oxygen atom and the heteroatoms, and more preferably 12 or less linking atoms. Preferably, the heteroatoms are oxygen, sulfur, nitrogen, phosphorus, boron and silicon atoms.

When the polymerizable photoinitiator (A) has a covalent bond between a heteroatom and a hydrogen atom, the functional group having the covalent bond may be OH, NH, SH, SiH, COOH, CONH, $CONH_2$, NHCOO, NHCONH, $SO_3H$, $PO_4H_2$ and the like are included. In addition, the functional group NHCOO (urethane bond) and the functional group NHCONH (urea bond) are preferred because hydrogens are more easily abstracted from the NH group under the influence of carbonyl (CO), and the initiation efficiencies of the polymerizable photoinitiator (A) become higher. As these functional groups of the polymerizable photoinitiator (A), any one type or two or more types selected from the above group, and one or two or more groups can be used. The number of one type of heteroatom-containing functional groups per molecule of the polymerizable photoinitiator (A) is preferably 1 to 40. When it has one or more heteroatom-containing functional groups, it can serve as a co-initiator derived from a heteroatom, which is preferable. On the other hand, when the number of the heteroatom-containing functional groups exceeds 40, since the hydrophilicity of the heteroatom-containing functional groups is high and hydrogen bonds between the functional groups are easily formed, there are problems that the hydrophilicity of the polymerizable photoinitiator (A) is lowered, the solubility in organic solvents is also lowered, and the water resistance and moisture resistance of the cured product of the curable composition containing the polymerizable photoinitiator (A) are lowered. From these viewpoints, the number of one type of heteroatom-containing functional groups per one molecule of the polymerizable photoinitiator (A) is more preferably 2 to 20, and particularly preferably 4 to 10.

The polymerizable photoinitiator (A) preferably further contains a structural unit derived from a polyol or a polyamine in the molecule. Since the benzophenone group of the polymerizable photoinitiator (A) has an aromatic planar structure and is strongly hydrophobic, it has low compatibility with polar (meth)acrylic monomers and oligomers, and depending on the composition of the active energy ray curable composition, a highly transparent curable composition and its cured product may not be obtained. Therefore, by introducing a urethane bond or urea bond as a polar functional group into the molecule of the polymerizable photoinitiator (A), the polymerizable photoinitiator (A) has both the rigidity derived from the aromatic group and the toughness derived from the urethane structure and urea structure, and has a good balance between hydrophilicity and hydrophobicity, and has good compatibility with general-purpose (meth)acrylic monomers and oligomers ranging from hydrophilic to hydrophobic, and a highly transparent active energy ray curable composition can be obtained by using such a polymerizable photoinitiator (A).

For the polymerizable photoinitiator (A), a method of introducing structural units derived from polyols or polyamines is not particularly limited. These structural units can be introduced by using polyols or polyamines as raw materials, and polyols are not particularly limited as long as they have two or more hydroxyl groups in the molecule, and polyamines are not particularly limited as long as they have two or more primary or secondary amino groups in the molecule. Examples of polyols include alkylene diols, alkylene polyols, polyether polyols, polyester polyols, polycarbonate polyols, polyolefin polyols, silicone polyols, and acrylic polyols. Examples of polyamines include alkylene diamines such as ethylenediamine, putrescine, cadaverine, triethylenediamine, and hexamethylenediamine, ethambutol, phenylenediamine, isophoronediamine, norbornenediamine, dicyclohexylmethanediamine, diethylenetriamine, bis(hexamethylene)triamine, spermine, spermidine, polyether polyamine, polyamide polyamine, polyamine epichlorohydrin, and polyethyleneimine. These polyols and polyamines may be used alone or in combination of two or more. Polyols are more preferred because the resulting cured product is less likely to be colored.

The polymerizable photoinitiator (A) can be produced using various known or common synthesis methods. For example, a method of reacting benzophenones having a hydroxyl groups with polymerizable compounds having isocyanate groups, carboxylic acid groups, or acid anhydride groups as functional groups; a method of sequentially or collectively urethanizing benzophenones having hydroxyl groups, polyisocyanates having two or more isocyanate groups in the molecule, and polymerizable compounds having hydroxyl groups; a method of sequentially or collectively urethanizing and/or ureating benzophenones, polyisocyanates, polyols and/or polyamines having hydroxyl groups and polymerizable compounds having hydroxyl groups; a method of directly reacting benzophenones with polymerizable compounds having hydroxyl groups, amino groups, glycidyl groups, etc., the benzophenones thereof having one or more carboxyl groups or acid anhydride functional groups as functional groups in the molecule, such as benzophenone dicarboxylic acid, benzophenone tetracarboxylic acid, and benzophenone tetracarboxylic dianhydride; a method of directly reacting benzophenones having one or more carboxyl groups or acid anhydride functional groups as functional groups in the molecule as described above with polyisocyanates, polyols, or polyamines as described above, etc., a method such as that described in Patent Literature 3 may also be used. In addition, in the manufacturing process, it is preferable to perform the work in an environment where light is blocked in order to suppress the generation of radicals due to light irradiation. Specifically, it is preferable to perform the reaction under shaded light, fluorescent light with no UV radiation, red darkroom safe light, or the like.

In the present embodiment, the molecular weight (number average) of the polymerizable photoinitiator (A) is preferably 1000 or more. When the molecular weight of the polymerizable photoinitiator (A) is 1000 or more, even if the polymerizable photoinitiator (A) remains in the cured product in an unreacted state or in the form of radicals formed by abstracting hydrogen, after the photopolymerization reaction (curing), since it is not a low molecular weight component with a molecular weight of less than 1000, it does not cause problems such as odor problems, bleed-out over time, coloring problems, and decreased durability of the cured product. As the molecular weight of the polymerizable photoinitiator (A) increases, the liquid viscosity of the active energy ray curable composition tends to increase, from the viewpoint of exhibiting good operability, the number average molecular weight of the polymerizable photoinitiator (A) is preferably 100,000 or less. Further, the number average molecular weight of the polymerizable photoinitiator (A) is more preferably 1,500 to 80,000, particularly preferably 2,000 to 50,000.

In the active energy ray curable composition of the present embodiment, the content of the polymerizable photoinitiator (A) is preferably 0.1 to 95% by mass based on the entire curable composition. When the polymerizable photoinitiator (A) is contained within this range, the function of generating radicals by irradiation with active energy rays such as ultraviolet rays, and the function of generating highly active free radicals by the subsequent hydrogen abstraction reaction are exhibited. In addition, from the viewpoint that by interaction with the polymerizable photoinitiator (A) and the polymerizable compound (B), the curability of the active energy ray curable composition can be improved and components with a molecular weight of less than 1000 in the obtained cured product can be reduced, the content of the polymerizable photoinitiator (A) is preferably 0.5 to 90% by mass, more preferably 1.0 to 80% by mass, based on the entire curable composition.

The active energy ray curable composition of the present embodiment has a polymerizable compound (B) (excluding A) having one or more ethylenically unsaturated groups per one molecule. The polymerizable compound (B) can be classified as monofunctional unsaturated compound (b1) having one ethylenically unsaturated group and one or more heteroatom-hydrogen covalent bonds, multifunctional unsaturated compound (b2) having two or more ethylenically unsaturated groups and one or more heteroatom-hydrogen covalent bonds, and monofunctional or multifunctional unsaturated compound (b3) having one or more ethylenically unsaturated group but no heteroatom-hydrogen covalent bonds. The ethylenically unsaturated groups of the polymerizable compound (B) are one or more groups selected from (meth)acrylamide group (meth)acrylate group, vinyl group, vinyl ether group, alkyl vinyl ether group, allyl group, (meth)allyl ether group, styryl groups, and maleimide group. These unsaturated compounds (b1), (b2) and (b3) may be used alone or in combination of two or more thereof. Furthermore, b1, b2, and b3 can be each independently used as a single compound selected from the same type or different types, or in any combination of a plurality of compounds.

The polymerizable compound (B) is preferably an unsaturated compound (b1) and/or (b2) having one or more covalent bonds between heteroatoms and hydrogen atoms. When the polymerizable compound (B) has one or more covalent bonds between heteroatoms and hydrogen atoms in the molecule thereof, B can serve as both a curable component and a co-initiator in the active energy ray curable composition. The polymerizable photoinitiator (A) abstracts a hydrogen atom from the covalent bond between the heteroatom and the hydrogen atom of the polymerizable compound (B), and the polymerizable compound (B) becomes a highly active free radical, which can initiate the radical polymerization of the ethylenically unsaturated groups of the polymerizable photoinitiator (A) and the polymerizable compound (B). In addition, since the radical of the polymerizable compound (B) formed by abstracting a hydrogen atom has higher activity than the radical of the polymerizable photoinitiator (A) formed by abstracting a hydrogen atom, the photopolymerization reaction, i.e., the curing reaction of the active energy ray curable composition can proceed at a higher rate with higher safety, the curing reaction can be completed even with low-energy long-wavelength light, and the content of low molecular weight components in the obtained cured product can be controlled to less than 10%. From these viewpoints, it is more preferable that the polymerizable compound (B) ((b1) and/or (b2)) has two or more covalent bonds of heteroatoms and hydrogen atoms per one molecule. The heteroatom is preferably at least one of an oxygen atom, a sulfur atom, a nitrogen atom, a phosphorus atom, a boron atom, and a silicon atom.

When the polymerizable compound (B) has a covalent bond between a heteroatom and a hydrogen atom, the functional group having the covalent bond may be OH, NH, SH, SiH, COOH, CONH, $CONH_2$, NHCOO, NHCONH, $SO_3H$, $PO_4H_2$, etc. In addition, the functional group NHCOO (urethane bond) and the functional group NHCONH (urea bond) are preferable because hydrogens are more easily abstracted from the NH groups under the influence of carbonyl (CO), and the initiation efficiency of the polymerizable photoinitiator (A) becomes higher, which is preferable. Further, as these functional groups of the polymerizable photoinitiator (A), any one or two or more types selected from the above group, or one or two or more groups can be used.

When the monofunctional unsaturated compound (b1) having a covalent bond between a hetero atom and a hydrogen atom has (meth)acrylate as an ethylenically unsaturated group, specific examples thereof include hydroxyalkyl (meth)acrylates into which a linear, branched, or cyclic hydroxyalkyl group having 1 to 18 carbon atoms has been introduced; (meth)acrylic acid ethyl carboxylic acid consisting of (meth)acrylic acid and hydroxyalkyl carboxylic acids; (meth)acrylic acid alkylcarboxylic acids such as (meth)acrylic acid ethylsuccinic acid, (meth)acrylic acid ethyl phthalic acid, (meth)acrylic acid ethylhexahydrophthalic acid; (meth)acrylic acid alkylsulfonic acids into which a linear, branched, or cyclic alkylsulfonic acid group having 1 to 18 carbon atoms has been introduced; (meth)acrylic acid alkyl phosphoric acids into which a linear, branched, or cyclic alkyl phosphoric acid group having 1 to 18 carbon atoms has been introduced; aminoalkyl (meth)acrylates into which an aminoalkyl group having 1 to 18 carbon atoms has been introduced; N-alkylaminoalkyl (meth)acrylates into which an N-alkylaminoalkyl group consisting of an aminoalkyl group having 1 to 18 carbon atoms and an alkyl group having 1 to 18 carbon atoms has been introduced; glycerin mono(meth)acrylate and the like. Among them, hydroxyalkyl (meth)acrylate, (meth)acrylic acid alkylcarboxylic acids and aminoalkyl (meth)acrylate are preferable as the monomer having a covalent bond between a heteroatom and a hydrogen atom.

When the monofunctional unsaturated compound (b1) having a covalent bond between a hetero atom and a hydrogen atom has a (meth)acrylamide group as an ethylenically unsaturated group, specific examples thereof include (meth)acrylamide; N-alkyl (meth)acrylamide into which a linear, branched, or cyclic alkyl group having 1 to 18 carbon atoms has been introduced; N-hydroxyalkyl (meth)acrylamide, N, N-di(hydroxyalkyl)(meth)acrylamide and N-hydroxyalkyl-N-(4-hydroxyphenyl)(meth)acrylamide into which a hydroxyalkyl group having 1 to 18 carbon atoms has been introduced; N-alkyl-N-hydroxyalkyl (meth)acrylamide and N-alkyl-N-(4-hydroxyphenyl) (meth)acrylamide into which a hydroxyalkyl group having 1 to 18 carbon atoms and an alkyl group having 1 to 18 carbon atoms have been introduced; 4-hydroxyphenyl (meth)acrylamide; N, N-di(4-hydroxyphenyl)(meth)acrylamide; (meth)acrylamidoalkylcarboxylic acid into which an alkylcarboxyl group having 1 to 18 carbon atoms has been introduced; N-alkoxyalkyl (meth)acrylamide into which an alkoxyalkyl group consisting of an alkoxy group having 1 to 18 carbon atoms and an alkylene group having 1 to 16 carbon atoms has been introduced; N-sulfoalkylacrylamide into which an alkylsulfonic acid group having 1 to 18 carbon atoms has been introduced; N-alkylamino(meth)acrylamide into which an aminoalkyl group having 1 to 18 carbon atoms has been introduced; N-alkylaminoalkyl (meth)acrylamide into which an N-alkylaminoalkyl group consisting of an aminoalkyl group having 1 to 18 carbon atoms and an alkyl group having 1 to 18 carbon atoms has been introduced; N, N-dialkylaminoalkyl (meth)acrylamide into which an N, N-dialkylaminoalkyl group consisting of an aminoalkyl group having 1 to 18 carbon atoms and an alkyl group having 1 to 18 carbon atoms has been introduced; diacetone acrylamide and the like. Among them, N-isopropyl acrylamide, N, N-dimethylaminopropyl acrylamide, N-hydroxyethyl acrylamide and diacetone acrylamide are preferable because they are easily available industrially, and as the monomer having a covalent bond between a hetero atom and a hydrogen atom, hydroxyalkyl (meth)acrylamide, (meth)acrylamidoalkyl carboxylic acid, aminoalkyl (meth)acrylamide, diacetone acrylamide, etc. are preferable. In particular, N-hydroxyethyl (meth)acrylamide, N-hydroxypropyl (meth)acrylamide, and N-hydroxybutyl (meth)acrylamide are more preferred because they are liquid at room temperature and do not cause skin irritation (P.I.I. = 0).

When the monofunctional unsaturated compound (b1) having a covalent bond between a hetero atom and a hydrogen atom has a vinyl group as an ethylenically unsaturated group, specific examples thereof include carboxylic acid vinyl esters into which a carboxylic acid having 1 to 22 carbon atoms has been introduced; maleic acid; fumaric acid; itaconic acid; maleic acid monoalkyl esters, maleic acid monoalkylamide, fumaric acid monoalkyl ester, fumaric acid monoalkyl amide, itaconic acid monoalkyl ester and itaconic acid monoalkyl amide into which a linear, branched, or cyclic alkyl group having 1 to 22 carbon atoms has been introduced; vinyl sulfonic acid, and vinyl phosphoric acid. Among these, maleic acid, fumaric acid, itaconic acid and the like are preferred because they are industrially easily available.

When the monofunctional unsaturated compound (b 1) having a covalent bond between a hetero atom and a hydrogen atom has an allyl group as an ethylenically unsaturated group, specific examples thereof include carboxylic acid allyl ester into which a carboxylic acid having 1 to 22 carbon atoms is introduced; allylamine; monoalkylallylamine into which a branched or cyclic alkyl group is introduced. In addition, when the monofunctional unsaturated compound (b 1) having a covalent bond between a hetero atom and a hydrogen atom has a styryl group as an ethylenically unsaturated group, specific examples thereof include p-styrenesulfonic acid with a sulfonic acid unit introduced therein. Furthermore, when the monofunctional unsaturated compound (b 1) having a covalent bond between a hetero atom and a hydrogen atom has a maleimide group as an ethylenically unsaturated group, specific examples thereof include N-hydroxyalkylmaleimide, N-(2-carboxyalkyl)maleimide, glycerin mono(N-hydroxyalkylmaleimide) ester, and trimethylolpropane mono(N-hydroxyalkylmaleimide) esters each having a linear, branched or cyclic alkyl group having 1 to 18 carbon atoms. Further, the various monofunctional unsaturated compounds (b 1) described above may be used alone or in combination of two or more.

As for the polyfunctional unsaturated compound (b2) having a covalent bond between a hetero atom and a hydrogen atom specific, examples include difunctional unsaturated compounds such as di(meth)acrylamide, diallylamine, and divinylamine, allyl (meth)acrylamide, alkyl diallylamine into which an alkyl group having 1 to 18 carbon atoms has been introduced, bisphenol A diglycidyl ether acrylic acid adducts, and polyurethane di(meth)acrylamides; further, trifunctional or higher polyfunctional unsaturated compounds such as pentaerythritol tri(meth)acrylate, dipentaerythritol tetra(meth)acrylate, dipentaerythritol penta(meth)acrylate, glycerin di(meth)acrylate, glycerin polyglycidyl ether poly(meth)acrylate, isocyanuric acid ethylene oxide modified tri(meth)acrylate, ethylene oxide modified dipentaerythritol penta(meth)acrylate, ethylene oxide modified pentaerythritol tri(meth)acrylate, and succinic acid-modified pentaerythritol tri(meth)acrylate. These polyfunctional unsaturated compounds (b2) may be used alone or in combination of two or more.

When the monofunctional or polyfunctional unsaturated compound (b3) having no covalent bond between a heteroatom and a hydrogen atom has one (meth)acrylate group as an ethylenically unsaturated group in the molecule, specific examples thereof include alkyl (meth)acrylates into which a linear, branched, or cyclic alkyl group having 1 to 18 carbon atoms has been introduced; alkoxyalkylene glycol (meth)acrylates, alkoxydialkylene glycol (meth)acrylates, alkoxytrialkylene glycol (meth)acrylates and alkoxypolyalkylene glycol (meth)acrylates, into which functional groups consisting of an alkyl group having 1 to 18 carbon atoms and an alkylene glycol group having 1 to 6 carbon atoms have been introduced; phenoxyalkylene glycol (meth)acrylates, phenoxydialkylene glycol (meth)acrylates, phenoxytrialkylene glycol (meth)acrylates and phenoxypolyalkylene glycol (meth)acrylates, into which a functional group consisting of a phenoxy group and an alkylene glycol group having 1 to 6 carbon atoms has been introduced; N, N-dialkylaminoalkyl (meth)acrylates into which an N, N-dialkylaminoalkyl group consisting of an aminoalkyl group having 1 to 18 carbon atoms and an alkyl group having 1 to 18 carbon atoms has been introduced; (meth)acrylates with a cyclic structure introduced such as benzyl (meth)acrylate, phenoxyethyl (meth)acrylate, dicyclopentanyl (meth)acrylate, dicyclopentanyloxyethyl (meth)acrylate, dicyclopentenyl (meth)acrylate, dicyclopentenyloxyethyl (meth)acrylate, bornyl (meth)acrylate, isobornyl (meth)acrylate, tetrahydrofurfuryl (meth)acrylate and 2-methyl-2-adamantyl (meth)acrylate; (meth)acrylates with epoxy groups introduced, such as glycidyl (meth)acrylate, and 4-hydroxybutyl (meth)acrylate glycidyl ether.

When the monofunctional or polyfunctional unsaturated compound (b3) having no covalent bond between a hetero atom and a hydrogen atom has one (meth)acrylamide group as an ethylenically unsaturated group in the molecule, specific examples thereof include N, N-dialkyl (meth)acrylamide into which a linear, branched, or cyclic alkyl group having 1 to 18 carbon atoms has been introduced; (meth)acryloylmorpholine; N, N-di(alkoxyalkyl)(meth)acrylamide into which an alkoxyalkyl group consisting of an alkoxy group having 1 to 18 carbon atoms and an alkylene group having 1 to 16 carbon atoms has been introduced; N-alkyl-N-alkoxyalkyl (meth)acrylamide into which an alkoxyalkyl group consisting of an alkoxy group having 1 to 18 carbon atoms and an alkylene group having 1 to 18 carbon atoms, and an alkyl group having 1 to 18 carbon atoms has been introduced; and N-alkyl-N-(dialkylamino)alkyl(meth)acrylamide into which a dialkylaminoalkyl group consisting of an alkyl group having 1 to 18 carbon atoms and an alkylene group having 1 to 18 carbon atoms, and an alkyl group having 1 to 18 carbon atoms has been introduced. Among them, N, N-dimethylacrylamide, N, N-diethylacrylamide, N-methyl-N-(dimethylamino)propylacrylamide, N-acryloylmorpholine, etc. are preferred because they are industrially easily available. Particularly preferred is N-acryloylmorpholine, which is liquid at room temperature and has low skin irritation (P.I.I. = 0.5).

When the monofunctional or polyfunctional unsaturated compound (b3) having no covalent bond between a hetero atom and a hydrogen atom has one vinyl group as an ethylenically unsaturated group in the molecule, specific examples thereof include alkyl vinyl ether into which a straight chain, branched, or cyclic alkyl group having 1 to 22 carbon atoms has been introduced, vinyl chloride, N-vinylpyrrolidone, N-vinylcaprolactam, N-vinyloxazoline, maleic anhydride, itaconic anhydride, maleic acid dialkyl ester, maleic acid dialkyl amide, maleic acid alkylimide, fumaric acid dialkyl ester, fumaric acid dialkyl amide, itaconic acid dialkyl ester, itaconic acid dialkyl amide, itaconic acid alkylimide, and vinyl carboxylic acid. Among them, N-vinylpyrrolidone, N-vinylcaprolactam, maleic anhydride and the like are preferred because they are industrially easily available.

When the monofunctional or polyfunctional unsaturated compound (b3) having no covalent bond between a hetero atom and a hydrogen atom has one allyl group as an ethylenically unsaturated group in the molecule, specific examples thereof include alkyl allyl ether into which a straight chain, branched, or cyclic alkyl group having 1 to 22 carbon atoms has been introduced, phenyl allyl ether, alkyl phenyl allyl ether, dialkylallylamine into which a branched or cyclic alkyl group is introduced. When the monofunctional or polyfunctional unsaturated compound (b3) having no covalent bond between a hetero atom and a hydrogen atom has one styryl group as an ethylenically unsaturated group in the molecule, specific examples thereof include styrene, $\alpha$-alkyl styrene into which a alkyl group having 1 to 18 carbon atoms has been introduced into $\alpha$-positions, $\alpha$-methylstyrene dimer, o-alkyl styrene, m-alkyl styrene and p-alkyl styrene, into which alkyl groups having 1 to 18 carbon atoms have been introduced into phenyl groups. Among them, styrene and $\alpha$-

methylstyrene, α-methylstyrene dimer are preferred because they are industrially easily available.

When the monofunctional or polyfunctional unsaturated compound (b3) having no covalent bond between a hetero atom and a hydrogen atom has two or more ethylenically unsaturated groups in the molecule, specific examples thereof include di(meth)acrylate, divinyl compounds such as divinylbenzene, bismaleimide compounds, allyl(meth)acrylate, methyl(2-allyloxymethyl)acrylate, 2-(2-vinyloxyethoxy)ethyl acrylate, onium salts formed by combining at least one anion selected from known inorganic acid anions or organic acid anions and a dialkyl diallylammonium cation into which an alkyl group having 1 to 18 carbon atoms has been introduced, alkylene glycol di(meth)acrylates, polyalkylene glycol di(meth)acrylates, alkoxylated bisphenol A diacrylates, polyester di(meth)acrylates, polycarbonate di(meth)acrylates, polyurethane di(meth)acrylates, pentaerythritol tetra(meth)acrylate, trimethylolpropane tri(meth)acrylate, dipentaerythritol tri(meth)acrylate, ditrimethylolpropane tetra(meth)acrylate, dipentaerythritol hexa(meth)acrylate, tri(meth)acryloyloxyethoxytrimethylolpropane, ethylene oxide-modified dipentaerythritol hexa(meth)acrylate, ethylene oxide-modified pentaerythritol tetra(meth)acrylate, and polyethoxy-modified trimethylolpropane tri(meth)acrylate. Further, the aforementioned various monofunctional or polyfunctional unsaturated compounds (b3) may be used alone or in combination of two or more.

In the active energy ray curable composition of the present embodiment, the content of the polymerizable compound (B) is with respect to the entire curable composition 5 to 99.9% by mass. When the content of the polymerizable compound (B) is within this range, the curability with respect to active energy rays such as ultraviolet rays is high, the curing reaction can be completed in a short time, the amount of remaining unreacted components is extremely small, and the content of low molecular weight components in the obtained cured product can be suppressed to a low level. The content of the polymerizable compound (B) is more preferably 10 to 99.5% by mass, and particularly preferably 20 to 99 % by mass.

The content of the monofunctional unsaturated compound (b1) having a covalent bond between a hetero atom and a hydrogen atom as a polymerizable compound (B) is 0 to 98% by mass based on the entire active energy ray curable composition. The content is preferably from 5 to 90% by mass, more preferably from 10 to 80% by mass. Further, the content of the polyfunctional unsaturated compound (b2) having a covalent bond between a hetero atom and a hydrogen atom is preferably 0 to 90% by mass based on the entire active energy ray curable composition, more preferably 1 to 80% by mass, particularly preferably 5 to 70% by mass. Furthermore, the content of the monofunctional or polyfunctional unsaturated compound (b3) that does not have a covalent bond between a hetero atom and a hydrogen atom is 0 to 90% by mass based on the entire active energy ray curable composition, more preferably 2 to 75% by mass, particularly preferably 5 to 65% by mass. b1 and b2 are compounds that have covalent bonds between heteroatoms and hydrogen atoms, while they are polymerizable compounds, they also function as hydrogen donors, therefore if their content is 3% by mass or more in total, the active energy ray curable composition can be completely cured with highly safe long wavelength light, and it is preferable because the cured product has a small amount of low molecular weight components, does not cause odor, bleed-out over time, or discoloration, and can obtain a cured product with high durability and safety. From the same viewpoint, it is more preferable that the total content of b1 and b2 is 6% by mass or more. If the contents of b1 and b2 are within these ranges, the active energy ray curable composition can be suitably used for various applications, such as inks, inkjet inks, photocurable three-dimensional modeling inks, two-dimensional or three-dimensional modeling inks, dental materials, nail cosmetics, pressure-sensitive adhesives, adhesives, sealing materials, coating agents, self-repairing paints, coating agents for vehicles, coating agents for building materials, and decorative films. Moreover, if the content of b1 to b3 is within these ranges, the polymerizable photoinitiator (A) and b 1 to b3 have good compatibility, an active energy ray curable composition having good transparency can be easily prepared, and the composition thereof can be suitably used for various applications in the optical field, such as optical pressure-sensitive adhesive sheets, optical adhesives, and optical sealants.

As the polymerizable compound (B), the monofunctional unsaturated compound (b 1) having a covalent bond between a hetero atom and a hydrogen atom may be used alone or in combination of two or more. As the polymerizable compound (B), the polyfunctional unsaturated compound (b2) having a covalent bond between a hetero atom and a hydrogen atom may be used alone or in combination of two or more. In addition, as the polymerizable compound (B), monofunctional or polyfunctional unsaturated compounds (b3) that do not have a covalent bond between a hetero atom and a hydrogen atom may be used alone or in combination of two or more. b1 to b3 can be used in appropriate combinations depending on the purpose. Among them, it is preferable to have a (meth)acrylamide group as the ethylenically unsaturated group. The amide bond of the (meth)acrylamide group has good wettability and adhesion to various substrates, and active energy ray curable compositions are suitably used in adhesives, paints, inks, and the like. In addition, due to the presence of amide bonds in the (meth)acrylamide group, intramolecular and intermolecular hydrogen bonds are easily formed, and the active energy ray curable composition has high cohesive strength, making it suitable for use as adhesives and sealing materials. Furthermore, the amide bond of (meth)acrylamide has higher acid resistance, alkyl resistance, and hydrolysis resistance than the ester bond of

(meth)acrylate, and when used as an ink for three-dimensional modeling, a dental material, coating agents for vehicles, coating agents for building materials, these cured products have excellent durability. Using hydroxyalkyl (meth)acrylamide as the monofunctional unsaturated compound (b 1) having a covalent bond between a hetero atom and a hydrogen atom and/or introducing the ethylenically unsaturated group of the polyfunctional unsaturated compound (b2) having a covalent bond between a hetero atom and a hydrogen atom using hydroxyalkyl (meth)acrylamide, even if unreacted b1 and b2 remain, high safety can be ensured, so use as a nail cosmetic is suitable. Furthermore, the use of methacrylamide groups tends to have higher heat resistance, water resistance, and moist heat resistance than when using acrylamide groups, on the other hand, the use of acrylamide groups tends to have higher curability and faster curing speed than the use of methacrylamide groups. It is preferable to appropriately select and use a monofunctional unsaturated compound (b 1) having a covalent bond between a (meth)acrylamide-based heteroatom and a hydrogen atom, polyfunctional unsaturated compound (b2) having a covalent bond between a heteroatom and a hydrogen atom, and a monofunctional or polyfunctional unsaturated compound (b3) that does not have a covalent bond between a hetero atom and a hydrogen atom according to various applications.

The active energy ray curable composition in the present embodiment can be used without containing an organic solvent. In addition, in order to improve workability such as coating properties, an organic solvent may be added as necessary to adjust the viscosity of the liquid. The added organic solvent may be removed in advance before curing at the time of active energy ray curing, or curing may be performed while the organic solvent is still contained. Further, the organic solvent may be removed after curing, and it can be appropriately selected according to the use method and purpose of the curable composition and the obtained cured product. The amount of the organic solvent to be added is not particularly limited, but is preferably 80% by mass or less and more preferably 50% by mass or less with respect to the total amount of the photocurable composition from the viewpoint of reducing energy and time required for removing the organic solvent.

Examples of the organic solvent used in the active energy ray curable composition of this embodiment include alcohols such as methanol, ethanol and isopropanol; ketones such as acetone, methyl ethyl ketone, methyl isobutyl ketone and cyclohexanone; esters such as ethyl acetate, propyl acetate, butyl acetate, methyl lactate and ethyl lactate; alkylene glycols such as ethylene glycol and propylene glycol; polyalkylene glycols such as polyethylene glycol and polypropylene glycol; glycol ethers such as ethoxydiethylene glycol and methoxypropylene glycol; glycol esters such as propylene glycol acetate; ethers such as tetrahydrofuran, methyltetrahydrofuran, cyclopentyl methyl ether, methyltetrahydropyran and methyl tert-butyl ether toluene; aromatic hydrocarbons such as xylene; aliphatic hydrocarbons such as hexane and cyclohexane; amides such as N, N'-dimethylformamide and dimethylacetamide; amide ethers such as β-methoxy-N, N-dimethylpropionamide and β-butoxy-N, N-dimethylpropionamide; pyrrolidones such as 2-pyrrolidone and N-methylpyrrolidone; piperidines such as N-methylpiperidine; halogenated hydrocarbons such as methylene chloride, chloroform, and dichloroethane; sulfoxides such as dimethyl sulfoxide; and imidazolidinones such as 1,3-dimethyl-2-imidazolidinone. These organic solvents may be used alone or in combination of two or more.

The light beam used for curing the active energy ray curable composition of the present embodiment is not particularly limited as long as upon irradiation, the polymerizable photoinitiator (A) can extract hydrogen atoms from the constituent components of the curable composition and generate active radicals. Specifically, visible light, electron beams, ultraviolet (vacuum ultraviolet, far ultraviolet, near ultraviolet), infrared (near infrared, mid-infrared, far infrared), laser light, infrared rays, X-rays, α-rays, β-rays, and γ-rays are mentioned, and it is preferable to use ultraviolet rays from the viewpoint of a good balance between the generation device, curing speed, and safety. One or more types selected from the group consisting of, for example, ultra-high pressure mercury lamp, high-pressure mercury lamp, low-pressure mercury lamp, mercury xenon lamp, metal halide lamp, high power metal halide lamp, xenon lamp, pulsed emission xenon lamp, deuterium lamp, fluorescent lamp, Nd-YAG3 harmonic laser lamp, He-Cd laser, nitrogen laser lamp, Xe-Cl excimer laser lamp, Xe-F excimer laser lamp, semiconductor pumped solid-state laser lamp, LED lamp can be used as the source (source) of the active energy ray. It is preferable from the viewpoint of high safety, energy saving, and environmental friendliness to use a light emitting diode (LED) lamp which generates ultraviolet rays having wavelengths of 350 to 420 nm, is highly efficient in converting energy into light, is easy to increase output, and does not use harmful mercury.

The active energy ray curable composition of the embodiment is suitably used in an active energy ray curable pressure-sensitive adhesive composition used for active energy ray curable pressure-sensitive adhesive, an active energy ray curable adhesive composition used for active energy ray curable adhesive, an active energy ray curable sealing material composition used for sealing material or caulking material, an active energy ray curable flexographic printing ink composition, an active energy ray curable offset printing ink composition, an active energy ray curable screen printing ink composition, an active energy ray curable inkjet printing ink composition, an active energy ray curable nail cosmetic composition used for gel nails, etc., an active energy ray curable dental compositions used for dentition models, orthodontics, implant treatments, etc., an active energy ray curable coating agent composition used in paints and coating agents for automobiles, electrical appliances, furniture, etc., an active energy ray curable decorative sheet composition used for decorative sheets as surface coating of automobiles, electrical appliances, etc., coating agent with self-repairing properties, compositions for active energy ray-curable self-repairing materials used for three-dimensional modeling objects, nail decoration materials, and automobile exterior protection, functional members such as decorative films, and devices, etc., active energy ray curable elastomer compositions used for elastomer materials used in transparent adhesive sheets, cushioning materials, packing, vibration-proofing materials, sound-absorbing materials, printing plates, sealing materials, abrasives, etc., active energy ray curable three-dimensional modeling compositions such as model materials and support materials for 3D printers, photocurable vehicle coating compositions such as automotive paints, etc.. Furthermore, the applications for which the active energy ray curable composition can be used are not limited to these. When the active energy ray curable composition is used for these applications, other components such as polymers and various additives may be mixed as necessary to adjust the composition according to the application.

Examples of the polymer to be added to the active energy ray curable composition include polyurethane resin, polyester resin, polyamide resin, polyimide resin, polyether resin, polyvinyl acetate, epoxy resin, polyacrylamide, rosins, starch, and carboxymethylcellulose. Among them, natural resins or processed resins derived from natural raw materials such as rosins, starches, carboxymethylcellulose, rosin-modified phenolic resin, rosin-modified maleic resin, rosin-modified alkyd resin, rosin-modified petroleum resin, rosin ester resin, and vegetable oil-modified alkyd resins are preferable because they have a high biomass degree. These polymers may be used alone or in combination of two or more kinds thereof. The amount of the polymer to be added is not particularly limited as long as it does not adversely affect the properties exhibited by the active energy ray curable composition and molded products for various uses using the same, it is preferably 10% by mass or less, more preferably 5% by mass or less, based on the entire active energy ray curable composition.

Examples of the additives added to the active energy ray curable composition include thermal polymerization inhibitors, antioxidants, ultraviolet sensitizers, preservatives, phosphate ester and other flame retardants, surfactants, antistatic agents, pigments such as yellow pigments, magenta pigments, cyan pigments, black pigments, and white pigments, dyes, perfumes, antifoaming agents, fillers, silane coupling agents, surface tension modifiers (surface modifiers), plasticizers, surface lubricants, leveling agents, softeners, organic fillers, inorganic fillers, and silica particles, and the like. These additives may be used alone or in combination of two or more kinds thereof. The content of these additives is not particularly limited as long as it does not adversely affect the properties exhibited by the active energy ray curable composition and molded articles for various uses using the same, it is preferably 30% by mass or less, more preferably 20% by mass or less, and particularly preferably 10% by mass or less based on the entire active energy ray curable composition.

The sensitizer added to the active energy ray curable composition is not particularly limited; however, anthracene sensitizers, thioxanthone sensitizers, and the like are preferable. Specific examples thereof include anthracene sensitizers such as 9,10-dibutoxy anthracene, 9,10-diethoxy anthracene, 9,10-dipropoxy anthracene, 9,10-bis(2-ethylhexyloxy) anthracene and thioxanthone sensitizers such as 2,4-diethyl thioxanthone, 2-isopropyl thioxanthone, 4-isopropyl thioxanthone. Typical examples of commercially available products include DBA, DEA (manufactured by Kawasaki Kasei Chemicals Co., Ltd) as anthracene sensitizers and DETX and ITX (manufactured by Lambson Co., Ltd) as thioxanthone sensitizers. Although the content of the sensitizing agent is not particularly limited, it is preferably 0.5% by mass or more, and more preferably 0.8% by mass or more based on the entire active energy ray curable composition. In addition, the content of the sensitizing agent is preferably 5.0% by mass or less, and more preferably 3.0% by mass or less based on the entire active energy ray curable composition. When the content of the sensitizing agent is within these ranges, the active energy ray curable composition has excellent curability, the resulting cured product has a low content of low molecular weight components, and has good durability and yellowing resistance.

EXAMPLES

Hereinbelow, the present invention is described still more specifically by showing Examples and Comparative Examples; however, the present invention is not limited to these Examples. In the following, all descriptions of "parts" and "%" are on a mass basis unless otherwise specified.

Table 1 shows the type and number of ethylenically unsaturated groups, the number of benzophenone groups, molecular weight, etc. of the polymerizable photoinitiators (A-1) to (A-18) used in Examples, and general-purpose photopolymerization initiators (D-1) to (D-4) used in Comparative Examples. In addition, (A-1) to (A-4) and (D-1) to (D-4) are commercially available products as follows, while (A-5) to (A-18) were synthesized by the method described in Patent Literature 3. A-1: 4-Methacryloyloxy benzophenone A-2: 2-Hydroxy-4-(acryloyloxy) benzophenone A-3: 2-Hydroxy-4-(methacryloylamino) benzophenone A-4: 2-Hydroxy-4-[2-(acryloylamino) ethoxy] benzophenone D-1: Benzophenone D-2: (1,1-Dimethyl-2-oxo-2-phenylethyl) acrylate D-3: Omnipol 910 (manufactured by IGM Resins, molecular weight: 1039) D-4: Speed Cure 7005 (manufactured by Lambson, molecular weight: 1300)

[Table 1]

| No. | Ethylenically unsaturated group | | Number of benzophenone groups | Heteroatom-containing functional group | | Number ratio per one molecule (Benzophenone group! Ethylenically unsaturated group) | Number of linked atoms between oxygen atom of ketones in benzophenone group and heteroatoms | Polyol, polyamine derived structures | Molecular weight |
| | Type (group) | Number (average) | | Type | Number (average) | | | | |
|---|---|---|---|---|---|---|---|---|---|
| A-1 | Methacrylate | 1 | 1 | - | - | 1 | - | - | 266 |
| A-2 | Acrylate | 1 | 1 | OH | 1 | 1 | 3 | - | 268 |
| A-3 | Methacrylamide | 1 | 1 | OH | 1 | 1 | 3 | - | 281 |
| | | | | CONH | 1 | | 5 | | |
| A-4 | Acrylamide | 1 | 1 | OH | 1 | 1 | 3 | - | 311 |
| | | | | CONH | 1 | | 8 | | |
| A-5 | Vinyl | 2 | 2 | - | - | 1 | - | Polyesterpolyol | 1200 |
| A-6 | Allyl | 10 | 2 | NH | 2 | 0.2 | 5 | Polyetherpolyol | 1150 |
| A-7 | Maleimide | 2 | 4 | NHCOO | 4 | 2 | 7 | Polycarbonate-polyol | 2940 |
| A-8 | Acrylate | 2 | 48 | OH | 2 | 8 | 4 | Polyesterpolyol Polyetherpolyol | 17400 |
| | Acrylamide | 4 | | NHCOO | 12 | | 25 | | |
| A-9 | Acrylate | 1 | 2 | NHCOO | 6 | 1 | 7 | Hexamethylenediamine | 3690 |
| | Vinyl | 1 | | NHCONH | 12 | | 10 | | |
| A-10 | Acrylamide | 6 | 2 | CONH | 6 | 0.14 | 15 | Polyesterpolyol | 10400 |
| | Allyl group | 8 | | NHCOO | 22 | | 4 | | |
| A-11 | Acrylamido | 2 | 6 | CONH | 2 | 2 | 27 | Polyetherpolyamine | 46900 |
| | Maleimide | 1 | | NHCOO | 15 | | 8 | | |
| | | | | NHCONH | 36 | | 15 | | |
| A-12 | Acrylamide | 2 | 2 | CONH | 2 | 0.5 | 8 | - | 989 |
| | Vinyl | 2 | | COOH | 4 | | 5 | | |

| No. | Ethylenically unsaturated group | | Number of benzophenone groups | Heteroatom-containing functional group | | Number ratio per one molecule (Benzophenone group! Ethylenically unsaturated group) | Number of linked atoms between oxygen atom of ketones in benzophenone group and heteroatoms | Polyol, polyamine derived structures | Molecular weight |
| | Type (group) | Number (average) | | Type | Number (average) | | | | |
|---|---|---|---|---|---|---|---|---|---|
| A-13 | Acrylate | 2 | 4 | NHCOO | 4 | 2 | 5 | Hexanediol | 11690 |
| A-14 | Methacrylamide | 2 | 2 | CONH | 2 | 1 | 60 | Polyetherpolyol | 3000 |
| | | | | NHCOO | 4 | | 48 | | |
| A-15 | Acrylamide | 2 | 6 | CONH | 430 | 3 | 5 | Polyamide-polyamine | 50400 |
| | | | | NHCONH | 12 | | 235 | | |
| A-16 | Methacrylate | 1 | 6 | COOH | 5 | 6 | 5 | Polyesterpolyol | 2180 |
| A-17 | Acrylamide | 4 | 30 | OH | 30 | 7.5 | 10 | Polycarbonate-polyol Polyamine epichlorohydrin | 15400 |
| | | | | CONH | 4 | | 29 | | |
| | | | | NHCOO | 10 | | 17 | | |
| | | | | NHCONH | 6 | | 10 | | |
| A-18 | Acrylamide | 1 | 12 | OH | 1 | 12 | 22 | Polyetherpolyol | 9660 |
| | | | | CONH | 1 | | 34 | | |
| | | | | NHCOO | 12 | | 22 | | |
| A-19 | Acrylamide | 12 | 1 | OH | 2 | 0.08 | 21 | Polyesterpolyol | 13700 |
| | | | | CONH | 12 | | 40 | | |
| | | | | NHCOO | 18 | | 21 | | |
| A-20 | Acrylamide | 2 | 4 | CONH | 2 | 2 | 15 | Ethylenediamine | 2840 |
| | | | | NHCOO | 10 | | 5 | | |
| | | | | NHCONH | 4 | | 10 | | |

(continued)

| No. | Ethylenically unsaturated group | | Number of benzophenone groups | Heteroatom-containing functional group | | Number ratio per one molecule (Benzophenone group! Ethylenically unsaturated group) | Number of linked atoms between oxygen atom of ketones in benzophenone group and heteroatoms | Polyol, polyamine derived structures | Molecular weight |
| --- | --- | --- | --- | --- | --- | --- | --- | --- | --- |
| | Type (group) | Number (average) | | Type | Number (average) | | | | |
| D-1 | - | - | 1 | - | - | - | - | - | 182 |
| D-2 | Acrylate | 1 | | - | - | - | - | - | 218 |
| D-3 | - | - | | - | - | - | - | Polyetherpolyol | 1039 |
| D-4 | - | - | 4 | - | - | - | - | Polyetherpolyol | 1300 |

The polymerizable compounds (B) and other components (C) used in Examples and Comparative Examples are shown below.

<Polymerizable Compounds (B)>

(1) Monofunctional unsaturated compound (b1) having a covalent bond between a heteroatom and a hydrogen atom

b1-1: 2-Hydroxyethylacrylamide (registered trademarks "Kohshylmer" and "HEAA", manufactured by KJ Chemicals Co., Ltd).
b1-2: N-isopropyl acrylamide (manufactured by KJ Chemicals Co., Ltd, registered trademarks "Kohshylmer" and "NIPAM")
b1-3: N-phenylacrylamide (manufactured by KJ Chemicals Co., Ltd, registered trademark "Kohshylmer")
b1-4: Dopamine (meth)acrylamide (manufactured by KJ Chemicals Co., Ltd, registered trademark " Kohshylmer ")
b1-5: 3-(Meth)acrylamide phenylboronic acid (manufactured by KJ Chemicals Co., Ltd., registered trademark "Kohshylmer")
b1-6: 3-Hydroxypropylmethacrylamide (manufactured by KJ Chemicals Corporation, registered trademark "Kohshylmer")
b1-7: Diacetone acrylamide (manufactured by KJ Chemicals Co., Ltd, registered trademark "Kohshylmer ")
b1-8: Methacrylic acid
b1-9: Hydroxyethyl acrylate
b1-10: 4-Hydroxybutyl acrylate
b1-11: N-methylacrylamide (manufactured by KJ Chemicals Co., Ltd, registered trademark "Kohshylmer")
b1 -12: 2-Methacryloyloxyethyl acid phosphate

(2) Polyfunctional unsaturated compound (b2) having a covalent bond between a heteroatom and a hydrogen atom

b2-1: Dipentaerythritol pentaacrylate
b2-2: Quick Cure8100 (UV-curable urethane oligomer (registered trademark "Quick Cure", manufactured by KJ Chemicals Co., Ltd).
b2-3: Diallyl amine
b2 4: Quick Cure7100 (UV-curable urethane oligomer (registered trademark "Quick Cure", manufactured by KJ Chemicals Corporation).
b2-5: Allylmethacrylamide
b2-6: UV-6640B (bifunctional urethane acrylate, manufactured by Mitsubishi Chemical Co., Ltd)
b2-7: Bisphenol-A epoxy acrylate oligomer (Miramer PE-210, manufactured by Miwon Specialty Chemical Co., Ltd)
b2-8: UV-3000B (bifunctional urethane acrylate, manufactured by Mitsubishi Chemical Co., Ltd)
b2-9:1,3-diallyloxy-2-propanol
b2-10: ethylenebisdiacrylamide

(3) Mono-or polyfunctional unsaturated compounds (b3) having no covalent bond between a heteroatom and a hydrogen atom

b3-1: Diethylacrylamide (manufactured by KJ Chemicals Co., Ltd, registered trademarks "Kohshylmer" and "DEAA")
b3-2: Acryloylmorpholine ("Kohshylmer" and "ACMO" registered trademarks, manufactured by KJ Chemicals Co., Ltd)
b3-3: Dimethylacrylamide (manufactured by KJ Chemicals Co., Ltd, registered trademarks "Kohshylmer" and "DMAA")
b3-4: 4-t-Butylcyclohexyl acrylate (manufactured by KJ Chemicals Co., Ltd, registered trademark "Kohshylmer")
b3-5: Isobornyl acrylate
b3-6: Tetrahydrofurfuryl acrylate
b3-7: Benzyl acrylate
b3-8: Ethyl carbitol acrylate (SR256, manufactured by Sartomer Co., Ltd)
b3-9: N-vinylcaprolactam

b3-10: Hexanediol diacrylate (manufactured by Kyoeisha Chemical Co., Ltd., Light Acrylate 1, 6HX-A)
b3-11: Ethoxylated (3) trimethylolpropane triacrylate (SR454, manufactured by Sartomer Co., Ltd)

<Other Components (C)>

C-1: Disproportionated rosins (polymer, biomass degree: 100%, trade name: dehydro abietic acid, manufactured by Fujifilm Wako Pure Chemical Industries, Ltd)
C-2: 2,4-Diethyl thioxanthone (sensitizer, manufactured by Lambson)
C-3: BYK-331 (leveling agent, polyether modified poly (dimethylsiloxane), manufactured by BYK Chemie Ltd)
C-4: NX-061 green (pigment dispersion, manufactured by Dainichiseika Co., Ltd)
C-5: Carbon black dispersion (manufactured by Mitsubishi Chemical Co., Ltd)
C-6: MEK-ST-40 (Coloydie silica dispersion, manufactured by Nissan Chemical Co., Ltd)
C-7: VALIFAST BLUE1613 (manufactured by Orient Chemical Industries Co., Ltd)
C-8: Reolosil QS-30 (manufactured by Tokuyama Co., Ltd)
C-9: Inorganic filler (titanium oxide)

Examples 1 to 24 and Comparative Examples 1 to 8 (preparation and evaluation of active energy ray curable compositions)

Using the polymerizable photoinitiator (A) or photopolymerization initiator (D), polymerizable compounds (B) and other components (C) shown in Table 1, various components were weighed in proportions shown in Tables 2 and 3, and mixed at 25°C for 30 minutes to obtain active energy ray curable compositions of Examples 1 to 24 and Comparative Examples 1 to 8. Using each of the obtained compositions, the compatibility, curability, content of low molecular weight components in the obtained cured product, and durability of the cured product were evaluated by the following methods, and the results are shown in Table 2 and Table 3.

<Compatibility Evaluation>

The state of the prepared active energy ray curable composition was visually observed, and the compatibility was evaluated in three stages.

O: It was in a completely dissolved and transparent state without any precipitate or turbidity.
△: It was found to have slight turbidity.
×: It was found to have precipitates or turbidity.

<Evaluation of Curability (365 nm, 385 nm and 405 nm)>

The obtained active energy ray curable composition was applied to an easy-adhesion treated surface of a PET film ("Cosmoshine A-4100" manufactured by Toyobo Co., Ltd) having a thickness of 100 $\mu$m using a bar coater so as to have a film thickness of 30 $\mu$m, and the coating film was cured by irradiation with ultraviolet rays. The cumulative amount of light at which the cured product was not tacked when touched was determined, and the curability was evaluated in four grades. The following three types of lamps 1) to 3) were used for ultraviolet irradiation. In addition, the lower the cumulative amount of light required until tack disappears (complete curing), the higher the curability.

1) UVLED lamp: wave 365 nm, illumination 1000 mW/cm$^2$
2) UVLED lamp: wave 385 nm, illumination 1000 mW/cm$^2$
3) UVLED lamp: wave 405 nm, illumination 1000 mW/cm$^2$

◎: When the cumulative amount of light was less than 1000 mJ/cm$^2$, there is no tack.
○: When the cumulative amount of light was 1000 mJ/cm$^2$ or more and less than 3000 mJ/cm$^2$, there is no tack.
△: When the cumulative amount of light was 3000 mJ/cm$^2$ or more and less than 20000 mJ/cm$^2$, there is no tack.
×: Tack remained even at the cumulative amount of light 20000 mJ/cm$^2$.

<Evaluation of Content of Low molecular weight Component in Cured Product>
The obtained active energy ray curable composition was applied to a heavy-release film (silicone-coated PET film) using a table-top roll laminator (RSL-382S, manufactured by Royal Sovereign) so to avoid air bubbles with a light-release separator (silicone-coated PET film) and to have a film thickness of 100 $\mu$m, and then irradiated with ultraviolet rays (UVLED lamp: wavelength 365 nm, integrated light quantity 10000 mJ/cm$^2$), three test pieces of size 5 cm$^2$ were cut out, dried at 90°C for 2 minutes, and weighed as a cured film for evaluating the content of low molecular weight components. The acetone 25 g and the cured film for evaluation of the content of low molecular weight components were placed in an ultraviolet-impermeable brown glass bottle, the glass bottle was sealed, and the glass bottle was rotated at 30°C for 48 hours to extract soluble components in the cured film. Thereafter, the acetone solution was filtered through a 0.45 $\mu$m filter, the content of low molecular weight components having a number-average molecular weight of less than 1,000 was determined by high performance liquid chromatography (HPLC), the content of low molecular weight components was calculated by the following formula, and evaluation was performed according to the following criteria.

content (%) = (Mass of extracted low molecular weight components/Mass of cured film before extraction) $\times$ 100%

◎: The content of low molecular weight components was less than 2%.
○: The content of low molecular weight components was 2% or more and less than 5%.
△: The content of low molecular weight components was 5% or more and less than 10%.
✕: The content of low molecular weight components was 10% or more.

<Evaluation of Durability of Cured Product>

A 75 $\mu$m thick heavy-release PET film (manufactured by Toyobo Co., Ltd., polyester film E7001) was brought into close contact with a horizontally placed glass plate, a spacer with the thickness of 1 mm and an internal size of 50 mm $\times$ 20 mm was placed, the active energy ray curable composition obtained in each of Examples and Comparative Examples was filled inside the spacer, a 50 $\mu$m-thick light-release PET film (manufactured by Toyobo Co., Ltd., polyester film E7002) was further placed thereon, and the active energy ray curable composition was cured by irradiation with ultraviolet rays (UVLED lamp: wave length 385 nm, light quantity 10000 mJ/cm$^2$). Thereafter, a cured product prepared by removing the release PET films on both sides was used as a test piece, and the test piece was allowed to stand for 168 hours in a thermo-hygrostat set at a temperature of 40°C and a humidity of 50% RH. The presence or absence of bleed-out and deformation on the surface of the test piece was visually observed and evaluated according to the following criteria.

◎: No bleed-out or deformation was observed.
○: Bleed-out or deformation was slightly observed.
△: Bleed-out and deformation were both slightly observed, or either is severely observed.
✕: The cured product became a viscous liquid.

[Table 2]

| Examples | Active energy ray curable composition | | | | | | | | | | Compa-tibility | Curabilty (wavelength in nm) | | | Content of low molecule component (%) | Durability |
|---|---|---|---|---|---|---|---|---|---|---|---|---|---|---|---|---|
| | Polymerizable photoinitiator (A) and the like | | Polymerizable compound (B) | | | | | | Others | | | | | | | |
| | | | Monofunctional (b1) | | Multi-functional (b2) | | Other (b3) | | | | | 365 | 385 | 405 | | |
| | Type | Mass (%) | Type | Mass (%) | Type | Mass (%) | Type | Mass (%) | Type | Mass (%) | | | | | | |
| 1 | A-1 | a | b1-1 | 10 | b2-1 | 25 | b3-1 | 33 | C-1 | 4 | ◎ | ○ | ○ | △ | △ | △ |
| | | | | | b2-2 | 20 | | | | | | | | | | |
| 2 | A-2 | 0.1 | b1-2 | 35 | b2-3 | 5 | b3-4 | 15 | C-2 | 4.9 | ◎ | △ | ○ | ○ | △ | △ |
| | | | | | b2-4 | 30 | b3-10 | 10 | | | | | | | | |
| 3 | A-3 | 0.5 | b1-3 | 20 | b2-5 | 30 | b3-2 | 40 | - | - | ◎ | ○ | ○ | ○ | ○ | ○ |
| | | | | | b2-6 | 9.5 | | | | | | | | | | |
| 4 | A-4 | 5 | b1-6 | 50 | b2-1 | 15 | b3-2 | 30 | - | - | ○ | ◎ | ◎ | ○ | ◎ | ○ |
| 5 | A-5 | 30 | b1-8 | 25 | b2-7 | 10 | b3-3 | 15 | - | - | ○ | ◎ | ◎ | ○ | ◎ | ○ |
| | | | b1-9 | 15 | b2-8 | 5 | | | | | | | | | | |
| 6 | A-6 | 10 | b1-4 | 5 | b2-6 | 20 | b3-5 | 20 | - | - | ◎ | ◎ | ◎ | ◎ | ◎ | ◎ |
| | | | | | | | b3-11 | 45 | | | | | | | | |
| 7 | A-7 | 20 | 61-2 | 25 | b2-1 | 47 | b3-7 | 5 | C-2 | 3 | ○ | ◎ | ◎ | ◎ | ○ | ○ |
| 8 | A-8 | 15 | b1-5 | 3 | b2-9 | 5 | - | - | C-1 | 10 | ○ | ◎ | ◎ | ◎ | ◎ | ◎ |
| | | | b1-9 | 27 | b2-8 | 40 | | | | | | | | | | |
| 9 | A-9 | 40 | b1-11 | 15 | b2-4 | 10 | b3-3 | 25 | - | - | ◎ | ◎ | ◎ | ◎ | ○ | ○ |
| | | | | | | | b3-6 | 10 | | | | | | | | |
| 10 | A-10 | 50 | b1-1 | 35 | b2-3 | 5 | b3-2 | 10 | - | - | ◎ | ◎ | ◎ | ◎ | ◎ | ◎ |
| 11 | A-11 | 60 | b1-6 | 10 | b2-5 | 5 | b3-4 | 20 | - | - | ◎ | ◎ | ◎ | ◎ | ◎ | ◎ |
| | | | | | | | b3-1 | 5 | | | | | | | | |
| 12 | A12 | 70 | b1-10 | 5 | b2-9 | 20 | b3-4 | 5 | - | - | ○ | ◎ | ◎ | ◎ | ◎ | ◎ |

(continued)

| Examples | Polymerizable photoinitiator (A) and the like | | Polymerizable compound (B) | | | | | | Others | | Compa -tibility | Curabilty (wavelength in nm) | | | Content of low molecule component (%) | Durability |
| | | | Monofunctional (b1) | | Multi-functional (b2) | | Other (b3) | | | | | | | | | |
| | Type | Mass (%) | Type | Mass (%) | Type | Mass (%) | Type | Mass (%) | Type | Mass (%) | | 365 | 385 | 405 | | |
| 13 | A-13 | 3 | b1-7 | 15 | b2-10 | 12 | b3-6 | 60 | C-3 | 0.1 | ◎ | ◎ | ◎ | ○ | ○ | ◎ |
| | | | b1-2 | 5 | b2-2 | 4.9 | | | | | | | | | | |
| 14 | A-14 | 80 | b1-1 | 7 | b2-6 | 10 | b3-2 | 3 | - | - | ◎ | ◎ | ◎ | ◎ | ◎ | ◎ |
| 15 | A-15 | 4 | - | - | b2-8 | 70 | b3-1 | 6 | - | - | ○ | ○ | ○ | ○ | ○ | Δ |
| | | | | | b2-1 | 20 | | | | | | | | | | |
| 16 | A-16 | 10 | - | - | - | - | b3-8 | 40 | C-2 | 10 | ○ | ○ | Δ | Δ | Δ | Δ |
| | | | | | | | b3-5 | 40 | | | | | | | | |
| 17 | A-17 | 8 | b1-3 | 10 | b2-4 | 12 | b3-3 | 65 | C-1 | 5 | ◎ | ◎ | ○ | ○ | ◎ | ◎ |
| 18 | A-18 | 2 | b1-2 | 45 | - | - | b3-10 | 3 | - | - | ◎ | ◎ | ○ | ○ | Δ | Δ |
| | | | b1-10 | 50 | | | | | | | | | | | | |
| 19 | A-18 | 9 | b1-9 | 40 | b2-5 | 1 | b3-2 | 50 | - | - | ◎ | ◎ | ◎ | ◎ | ○ | ○ |
| 20 | A-1 | 1 | b1-7 | 30 | b2-10 | 2 | - | - | - | - | ◎ | ◎ | ◎ | ◎ | ◎ | ◎ |
| | A-6 | 2 | b1-10 | 65 | | | | | | | | | | | | |
| 21 | A-2 | 34 | - | - | b2-1 | 2 | b3-2 | 3 | - | - | ○ | ◎ | ◎ | ◎ | ○ | ○ |
| | A-6 | 2 | | | | | | | | | | | | | | |
| 22 | A-13 | 30 | b1-6 | 15 | b2-6 | 10 | b3-3 | 15 | - | - | ◎ | ◎ | ◎ | ◎ | ◎ | ◎ |
| | A-18 | 30 | | | | | | | | | | | | | | |
| 23 | A-17 | 3 | b1-2 | 10 | b2-8 | 30 | b3-10 | 50 | C-1 | 5 | ◎ | ◎ | ○ | ○ | ○ | ○ |
| | | | | | | | | | C-2 | 2 | | | | | | |
| 24 | A-8 | 3 | b1-10 | 5 | b2-1 | 60 | - | - | - | - | ◎ | ◎ | ◎ | ◎ | ◎ | ◎ |
| | A-11 | 2 | | | b2-2 | 30 | | | | | | | | | | |

[Table 3]

| Comparative Examples | Active energy ray curable composition | | | | | | | | | | Compatibility | Curability (wavelength in nm) | | | Content of low molecule component (%) | Durability |
|---|---|---|---|---|---|---|---|---|---|---|---|---|---|---|---|---|
| | Polymerizable photoinitiator (A) and the like | | Polymerizable compound (B) | | | | | | Others | | | 365 | 385 | 405 | | |
| | | | Monofunctional (b1) | | Multifunctional (b2) | | Other (b3) | | | | | | | | | |
| | Type | Mass (%) | Type | Mass (%) | Type | Mass (%) | Type | Mass (%) | Type | Mass (%) | | | | | | |
| 1 | D-1 | 5 | b1-10 | 20 | b2-8 | 30 | b3-2 | 30 | C-1 | 5 | ◎ | Δ | × | × | × | × |
| | | | | | | | b3-10 | 10 | | | | | | | | |
| 2 | D-2 | 10 | - | - | b2-6 | 40 | b3-5 | 45 | C-2 | 5 | ◎ | ○ | Δ | × | × | × |
| 3 | D-1 | 2 | b1-9 | 20 | b2-8 | 35 | b3-3 | 40 | - | - | ◎ | ○ | Δ | × | Δ | × |
| | D-2 | 3 | | | | | | | | | | | | | | |
| 4 | D-3 | 5 | b1-10 | 20 | b2-1 | 30 | b3-5 | 40 | C-2 | 5 | ○ | ○ | Δ | × | × | × |
| 5 | D-3 | 30 | b1-10 | 10 | b2-8 | 40 | b3-5 | 20 | - | - | Δ | ◎ | ○ | Δ | × | × |
| 6 | D-4 | 5 | b1-10 | 20 | b2-1 | 30 | b3-2 | 40 | C-2 | 5 | Δ | Δ | × | × | × | × |
| 7 | D-4 | 30 | b1-10 | 10 | b2-8 | 40 | b3-5 | 20 | - | - | × | Δ | × | × | × | × |
| 8(*) | A-1 | 90 | - | - | - | - | - | - | C-2 | 10 | ◎ | ○ | Δ | × | × | × |

(*) Since A-1 and C-2 are both solids, 50% by mass of ethyl acetate was used as a solvent when preparing Comparative Example 8. In each evaluation, after coating, the cured product was dried at 80°C for 2 minutes to remove the solvent, and then UV irradiation was performed.

As is apparent from the results shown in Tables 2 and 3, the active energy ray curable compositions of the respective Examples have good compatibility and high curability against 365 nm, 385 nm and 405 nm rays, and even when cured using a highly safe UVLED lamp, the content of low molecular weight components in the cured product is extremely low, and cured products capable of providing both safety and durability can be obtained. On the other hand, in Comparative Example 1 in which a hydrogen abstraction type benzophenone (D-1) was used as a photopolymerization initiator, Comparative Example 2 in which a polymerized acrylate and a photopolymerization initiator (D-2) having an intramolecular cleavage type acrylate acetophenone were used, and Comparative Example 3 in which both D-1 and D-2 were used, it is possible to adjust the curability with respect to light rays of the three wavelengths to some extent by combining the photopolymerization initiators or increasing their content, but the curability with respect to long-wavelength light rays of 385 nm and 405 nm is not satisfactory. The cured products obtained in Comparative Examples 1 to 3 all had a high content of low molecular weight components, and had low safety and low durability. The photopolymerization initiator (D-2) has an acrylate as a polymerizable functional group, but a decomposition product of the same mole is generated simultaneously with the generation of an activity radical, and therefore the decomposition product remains in a cured product as a low molecular weight component, and the durability of the cured product is low. Further, in Comparative Examples 4 and 5 using the intramolecular cleavage type oligomer (D-3) as the photopolymerization initiator, although the curability was observed at 365 nm, when the content of D-3 is small (Comparative Example 4), the curability on the long-wavelength side of 385 nm and 405 nm is largely lowered, and when the content is large (Comparative Example 5), the compatibility is lowered. The photopolymerization initiator (D-3) is a photopolymerization initiator having a molecular weight of 1000 or more, but is an intramolecular cleavage type, so that a low molecular weight component is generated along with generation of a radical. As a result, the content of the low molecular weight component in the cured product was high, and the durability of the cured product was low. In Comparative Example 6,7 in which the hydrogen abstracting polymer (D-4) was used as the photopolymerization initiator, the curability was low at all wavelengths of 365 nm, 385 nm and 405 nm, and in Comparative Example 7 in which the content of D-4 was increased, but the curability was not improved, further the compatibility of the curable composition was poor, a large amount of low molecular weight components remained in the cured product, and the durability of the cured product was also poor. In Comparative Example 8, (A-1) was used as a polymerizable photoinitiator, but the polymerizable compounds (B) were not contained, and therefore, the curability with respect to light rays of each wavelength was low, and particularly, the durability of the obtained cured product was extremely low. As described above, it is considered that the different properties of these Examples and Comparative Examples are due to the interaction between polymerizable photoinitiator (A) and polymerizable compound (B) used in the active energy ray curable composition of the present invention.

Examples 25 to 36 and Comparative Examples 9 to 12 (Preparation and evaluation of active energy ray curable ink compositions)

The active energy ray curable composition obtained in Table 2, the polymerizable photoinitiator (A) or the photopolymerization initiator (D) shown in Table 1, and other components were weighed according to the proportions shown in Table 4 (solid content conversion), and uniformly mixed at room temperature to prepare an ink composition. Using the prepared ink composition, the viscosity was measured by the following method, and the pigment dispersibility in the case of containing a pigment dispersion was evaluated. The active energy ray curability of the ink composition and the surface drying property of the obtained cured film were evaluated, inkjet printing was further performed, and the ink ejection stability and the clearness of the printed matter were evaluated as printability. The results of these evaluations are shown in Table 4.

<Viscosity Measurement and Evaluation>

The viscosity of the ink composition was measured in accordance with JIS K5600-2-3 using a cone-plate viscosimeter (RE550 viscosimeter manufactured by Toki Sangyo Co., Ltd). The viscosity of the ink composition for ink jet printing was evaluated in four grades as follows.

⊚: less than 5 to 100 mPa· s
○: less than 100 to 500 mPa · s
△: less than 500 to 2000 mPa · s
✕: 2000 mPa · s or more

<Evaluation of Pigment Dispersibility>

Using the prepared ink composition, the state of aggregation or precipitation of the pigment was visually observed immediately after preparation and after standing for 2 months, and the pigment dispersibility was evaluated in four grades as follows.

©: Neither aggregation nor precipitation of the pigment was observed immediately after preparation nor after standing for 2 months.

O: No precipitation was observed immediately after preparation, but slight precipitation of the pigment was observed after standing for 2 months.

△: Slight aggregation and/or precipitation of the pigments was observed immediately after the preparation, and it was clearly observed after standing for 2 months.

×: Aggregation and/or precipitation of pigments were clearly observed even immediately after preparation.

Method for producing printed matter by ultraviolet irradiation

The obtained ink composition was applied to a PET film having a thickness of 100 $\mu$m by a bar coater (RDS12) (film thickness after drying: 20 $\mu$m), and cured by ultraviolet ray irradiation (UVLED lamp: wave 385 nm, illumination 1000 mW/cm$^2$) to produce a printed matter.

<Evaluation of Curability of Ink Composition>

When the printed matter was produced by the above method, the cumulative amount of light until the ink composition was completely cured (non-sticky state) was measured, and the curability was evaluated according to the following criteria.

◎: completely cured at 1000 mJ/cm$^2$
○: completely cured at 1000 to 2000 mJ/cm$^2$
△: completely cured at 2000 to 5000 mJ/cm$^2$
×: 5000 mJ/cm$^2$ or more is required until complete curing

<Evaluation of Surface Drying Property>

The printed matter produced by the above method was allowed to stand in an environment at a room temperature of 23°C and a relative atmospheric humidity of 50% for 5 minutes, high-quality paper was placed on the printed surface, a load of 1 kg/cm$^2$ was applied for 1 minute, and the degree of transfer of ink to the paper was evaluated according to the following criteria.

◎: The ink dried and there was no transfer to the paper.
○: The ink dried and there was slight transfer to the paper.
△: The ink was almost dry and there was transfer to paper.
×: The ink was hardly dried, and the transfer to paper was large.

Inkjet printing and printability evaluation

The prepared ink composition was filled in a commercially available inkjet printer (LuxelJet UV350GTW, manufactured by FUJIFILM Co., Ltd.), a solid image was printed using coated paper, and the printability of the ink was evaluated by the following method.

<Evaluation of Ejection Stability>

The printed state of the obtained printed matter was visually observed and evaluated according to the following criteria.

◎: Good printing was performed without nozzle omission.
○: Slight nozzle omission was observed.
△: There is nozzle omission in a wide range.
×: There is non-ejection.

<Sharpness Evaluation>

The image clarity of the printed matter obtained from the ink composition containing the pigment was visually observed and evaluated according to the following criteria.

◎: Ink bleeding was not observed at all, and the image was clear.
○: There was almost no ink bleeding, and the image was good.
△: Ink bleeding was slightly observed.
×: Significant ink bleeding was observed.

[Table 4]

| | No. | Ink composition — Curable composition or photoinitiator Type | Mass (%) | Others Type | Others Mass (%) | Viscosity | Pigment dispersibility | Curability | Surface drying property | Ejection stability | Sharpness |
|---|---|---|---|---|---|---|---|---|---|---|---|
| Examples | 25 | Example 4 | 99 | C-4 | 1 | ◎ | ◎ | ◎ | ◎ | ◎ | ◎ |
| | 26 | Example 6 | 65 | b3-2 | 30 | ◎ | ◎ | ◎ | ◎ | ◎ | ◎ |
| | | | | C-5 | 5 | | | | | | |
| | 27 | Example 9 | 40 | b3-2 | 10 | ◎ | - | ◎ | ◎ | ◎ | - |
| | | | | b3-10 | 50 | | | | | | |
| | 28 | Example 21 | 5 | b1-9 | 20 | ◎ | ○ | ◎ | ◎ | ◎ | ○ |
| | | | | b2-8 | 10 | | | | | | |
| | | | | b3-3 | 55 | | | | | | |
| | | | | C-4 | 10 | | | | | | |
| | 29 | Example 17 | 100 | - | - | ◎ | - | ○ | ◎ | ◎ | - |
| | 30 | Example 1 | 10 | b1-3 | 10 | ◎ | - | ◎ | ◎ | ◎ | - |
| | | Example 18 | 30 | b3-2 | 30 | | | | | | |
| | 31 | A-12 | 20 | b1-10 | 20 | ◎ | ◎ | ◎ | ○ | ◎ | ○ |
| | | | | b2-7 | 38 | | | | | | |
| | | | | b3-7 | 20 | | | | | | |
| | | | | C-5 | 2 | | | | | | |
| | 32 | A-13 | 5 | b3-2 | 50 | ◎ | ◎ | ○ | ○ | ◎ | ○ |
| | | | | b3-10 | 42 | | | | | | |
| | | | | C-6 | 3 | | | | | | |
| | 33 | A-16 | 2 | b1-11 | 20 | ◎ | - | ○ | ◎ | ◎ | - |
| | | | | b2-6 | 20 | | | | | | |
| | | | | b3-3 | 30 | | | | | | |
| | | | | b3-5 | 28 | | | | | | |

(continued)

| | | Ink composition | | | | Viscosity | Pigment dispersibility | Curability | Surface drying property | Ejection stablity | Sharpness |
|---|---|---|---|---|---|---|---|---|---|---|---|
| | | Curable composition or photoinitiator | | Others | | | | | | | |
| | | Type | Mass (%) | Type | Mass (%) | | | | | | |
| | 34 | Example 19 | 40 | b3-8 | 20 | ◎ | ◎ | ◎ | ◎ | ◎ | ◎ |
| | | | | b3-10 | 35 | | | | | | |
| | | | | C-2 | 1 | | | | | | |
| | | | | C-4 | 4 | | | | | | |
| | 35 | Example 12 | 25 | b3-9 | 30 | ◎ | - | ○ | ◎ | ◎ | - |
| | | | | b3-11 | 42 | | | | | | |
| | | | | C-7 | 3 | | | | | | |
| | 36 | Example 12 | 30 | b3-6 | 60 | ○ | ◎ | ○ | ◎ | ◎ | ◎ |
| | | A-11 | 5 | C-5 | 5 | | | | | | |
| Comparative Examples | 9 | Comparative Example 1 | 80 | b3-6 | 20 | ○ | - | × | × | ○ | - |
| | 10 | Comparative Example 2 | 60 | b3-3 | 20 | Δ | × | ○ | Δ | × | × |
| | | | | b3-10 | 15 | | | | | | |
| | | | | C-5 | 5 | | | | | | |
| | 11 | Comparative Example 5 | 40 | b2-1 | 30 | Δ | - | Δ | × | × | - |
| | | | | b3-6 | 30 | | | | | | |
| | 12 | Comparative Example 7 | 40 | b2-8 | 10 | × | × | Δ | × | × | × |
| | | | | b3-1 | 45 | | | | | | |
| | | | | C-4 | 5 | | | | | | |

The viscosity of the active energy ray curable ink composition of the present invention can be arbitrarily adjusted according to various printing methods such as inkjet printing, offset printing, screen printing, and flexible printing. As is clear from the results in Table 4, the liquid viscosity can be adjusted to be low as an ink composition for inkjet printing, and when a pigment is blended, the ink composition has high pigment dispersibility. The reason why such results can be obtained is that the compatibility between the polymerizable photoinitiator (A) and the polymerizable compound (B) contained in Examples is extremely good, and it is easy to combine from a monofunctional unsaturated compound (b1) or (b3) having a low viscosity to a multifunctional unsaturated compound (b2) or (b3) having a high viscosity. In addition, due to the combination of the polymerizable photoinitiator (A) and the polymerizable compound (B), the ink compositions of the Examples had high curability, and since the amount of low molecular weight components in the obtained cured films was small, the surface drying properties of the cured films were good, and the ejection stability of the ink, which is printability as an inkjet ink composition, and the clarity of printed matter were good. On the other hand, all of the ink compositions of Comparative Examples were insufficient in any one or more of viscosity, pigment dispersibility, curability, surface drying property, and ejection stability, and were not satisfactory in inkjet printability.

Examples 37 to 45 and Comparative Examples 13 to 15 (Preparation and evaluation of active energy ray curable ink composition for three-dimensional modeling)

The active energy ray curable composition obtained in Table 2, the polymerizable photoinitiator (A) or the photopolymerization initiator (D) shown in Table 1, and other components were weighed according to the proportions shown in Table 5 (solid content conversion), and uniformly mixed at room temperature to prepare an ink composition for three-dimensional modeling. Using the ink composition for three-dimensional modeling, a three-dimensional modeled object was produced by the following method, and the curing shrinkage ratio during modeling was evaluated, and the strength, heat resistance, water resistance, and shaping accuracy of the obtained cured product were evaluated. The evaluation results are shown in Table 5.

<Strength Evaluation>

A 75 $\mu$m-thick heavy-release PET film (manufactured by Toyobo Co., Ltd., polyester film E7001) was brought into close contact with a horizontally placed glass plate, a spacer with the thickness of 1 mm and punched out into a No. 2 dumbbell shape inside conforming to JIS K6251 was placed, each of the three dimensional modeling ink compositions obtained in Examples and Comparative Examples was filled inside the spacer, a 50 $\mu$m-thick light-release PET film (manufactured by Toyobo Co., Ltd., polyester film E7002) was further placed thereon, and was cured by irradiation with ultraviolet rays from both sides (UVLED lamp: wave 365 nm, illumination 10 mW/cm$^2$, cumulative light quantity 5000 mJ/cm$^2$) to cure the three dimensional modeling ink composition. Thereafter, the release PET films on both sides were removed to obtain test pieces of cured products for Examples and Comparative Examples. In accordance with JIS K7161, tensile strength was measured using a table-top precision universal tester (Autograph AGS-X, manufactured by Shimadzu Co., Ltd.) under conditions of a tensile speed of 10 mm/min and a chuck-to-chuck distance of 50 mm in a temperature environment of 25°C, and strength was evaluated according to the following criteria.

◎: Tensile strength was 40 MPa or more.
○: Tensile strength was 30 MPa or more and less than 40 MPa.
△: Tensile strength was 20 MPa or more and less than 30 MPa.
×: Tensile strength was less than 20 MPa.

<Resistance Cure Shrinkage Evaluation>

The curing shrinkage rate was determined according to JIS K5600 2-4 based on the density change before and after curing of the ink composition for three-dimensional modeling as shown in the following calculation formula (1). The density of the three-dimensional modeling ink composition before and after curing was measured using an electronic hydrometer (MDS-300 manufactured by Alpha Mirage Co., Ltd.) in accordance with JIS K7112. The cured product was prepared in the same manner as the test piece for the tensile test. The following evaluations were made from the obtained curing shrinkage rates.

$$\text{Curing shrinkage rate (\%)} = (Ds - Dl) / Dl \times 100\% \quad \text{formula (1)}$$

(In the formula, Ds is the density of the ink composition for three-dimensional modeling after curing, and Dl is the density of the ink composition for three-dimensional modeling before curing.)

◎: Cure shrinkage was less than 6%.
○: Cure shrinkage was 6% or more and less than 7%.
△: Cure shrinkage was 7% or more and less than 8%.
×: Cure shrinkage was 8% or more.

<Heat Resistance Evaluation>

A cured product was prepared in the same manner as the test piece for the tensile test, and the glass transition temperature (Tg) of the cured product was measured using a differential scanning calorimeter (DSC-60plus, manufactured by Shimadzu Co., Ltd.). The heat resistance was evaluated based on the measured value of the glass transition temperature (Tg) of the cured product according to the following criteria.

©: The Tg of the cured product was 60°C or higher.

The Tg of the cured product was 40°C or more and less than 60°C.

×: The Tg of the cured product was less than 40°C.

<Water Resistance Evaluation>

A 75 $\mu$m-thick heavy-release PET film (manufactured by Toyobo Co., Ltd., polyester film E7001) was brought into close contact with a horizontally placed glass plate, a spacer with the thickness of 10 mm and an internal bottom surface size of 10 cm × 1 cm was placed, and each of the ink compositions for three-dimensional modeling obtained in Examples and Comparative Examples was filled inside the spacer corresponding to the 1 mm thickness, after smoothing the surface by keeping it warm at 60°C for 30 seconds, the ink composition for three-dimensional modeling was cured by irradiation with ultraviolet rays (UVLED lamp: wavelength 365nm, illuminance 10 mW/cm$^2$, cumulative light amount 5000 mJ/cm$^2$) to obtain a cured product with the length of 10 cm, the width of 1 mm and the thickness of 1mm. After measuring the weight of the obtained cured product immediately after production, it was immersed in a beaker containing 100 ml of water, and one day later, the weight after immersion was measured. The water absorption rate was measured by substituting the weight immediately after production and the weight after immersion into the following formula, and the water resistance was evaluated based on the criteria shown below.

Water absorption rate (%) = (Weight after one day-weight immediately after manufacture)/Weight immediately after manufacture × 100%

◎: Water absorption was less than 2%.
○: Water absorption rate was 2% or more and less than 2.5%.
△: Water absorption rate was 2.5% or more and less than 3%.
×: Water absorption rate was 3% or more.

<Shaping Accuracy Evaluation>

A 75 $\mu$m-thick heavy-release PET film (manufactured by Toyobo Co., Ltd., polyester film E7001) was brought into close contact with a horizontally placed glass plate, a spacer with the thickness of 10 mm and an internal size of 10 mm × 10 mm was placed, and each of the ink compositions for three dimensional modeling obtained in Examples and Comparative Examples was filled inside the spacer corresponding to the 1 mm thickness. Then, the surfaces were smoothed by keeping at 60°C for 30 seconds, and the ink compositions for three-dimensional modeling were cured by irradiation with ultraviolet rays (UVLED lamp: wave length 365 nm, illumination 10 mW/cm$^2$, cumulative light quantity 1000 mJ/cm$^2$). Thereafter, the three-dimensional modeling ink composition was filled to a 1 mm thickness and cured 10 times in total to obtain a cured product of 10 × 10 × 10 mm$^3$. The height of the obtained cured product was measured. Further, the side surface of the obtained cured product was visually observed. These results were combined, and the modeling accuracy was evaluated based on the following criteria.

◎: The height was less than 10 mm ± 0.1 mm, and there was no unevenness on the side surface.

○: The height was 10 mm ± 0.1 mm or more and less than ± 0.2 mm, or these was slight unevenness on the side surface.

△: The height was 10 mm ± 0.2 mm or more and less than ± 0.3 mm, or these was some unevenness on the side surface.

×: The height was 10 mm ± 0.3 mm or more, or these was obvious unevenness on the side surface.

[Table 5]

| | | ink composition for three-dimensional modeling | | | | Resistance cure shrinkage | Heat resistance | Water resistance | Strength | Shaping accuracy |
|---|---|---|---|---|---|---|---|---|---|---|
| | | Curable composition | | Others | | | | | | |
| | | Type | Mass (%) | Type | Mass (%) | | | | | |
| Examples | 37 | Example 5 | 70 | b1-6 | 25 | ◎ | ◎ | ◎ | ◎ | ○ |
| | | | | C-4 | 5 | | | | | |
| | 38 | Example 1 | 40 | b2-2 | 20 | ◎ | ◎ | ◎ | ◎ | ◎ |
| | | Example 11 | 40 | | | | | | | |
| | 39 | Example 22 | 50 | b2-4 | 10 | ◎ | ◎ | ◎ | ○ | ◎ |
| | | | | b3-5 | 40 | | | | | |
| | 40 | Example 14 | 100 | - | - | ◎ | ◎ | ◎ | ○ | ◎ |
| | 41 | Example 23 | 40 | b3-4 | 55 | ◎ | ○ | ◎ | ◎ | ○ |
| | | A-11 | 5 | | | | | | | |
| | 42 | A-7 | 30 | b2-7 | 40 | ◎ | ◎ | ◎ | ◎ | ○ |
| | | | | b3-2 | 30 | | | | | |
| | 43 | Example 10 | 20 | b2-6 | 30 | ◎ | ◎ | ◎ | ○ | ◎ |
| | | | | b3-2 | 20 | | | | | |
| | | | | b3-4 | 30 | | | | | |
| | 44 | A-13 | 3 | b1-7 | 20 | ◎ | ◎ | ○ | ○ | ◎ |
| | | | | b3-3 | 40 | | | | | |
| | | A-8 | 2 | b2-4 | 10 | | | | | |
| | | | | b2-8 | 25 | | | | | |
| | 45 | A-18 | 50 | b1-6 | 5 | ○ | ◎ | ○ | ○ | ○ |
| | | | | b2-1 | 30 | | | | | |
| | | | | b3-6 | 15 | | | | | |

EP 4 424 719 A1

EP 4 424 719 A1

(continued)

| | | ink composition for three-dimensional modeling | | | | Resistance cure shrinkage | Heat resistance | Water resistance | Strength | Shaping accuracy |
|---|---|---|---|---|---|---|---|---|---|---|
| | | Curable composition | | Others | | | | | | |
| | | Type | Mass (%) | Type | Mass (%) | | | | | |
| Comparative Examples | 13 | Comparative Example 3 | 20 | b2-1 | 40 | × | × | × | △ | × |
| | 14 | Comparative Example 4 | 100 | - | - | × | ○ | × | △ | × |
| | 15 | Comparative Example 6 | 80 | b3-5 | 20 | × | × | × | × | × |

31

As is clear from the results in Table 5, three-dimensional modeling using the ink compositions for three-dimensional modeling of Examples, since both the polymerizable photoinitiator (A) and the polymerizable compounds (B) have polymerizability, a modeled object having sufficient strength, water resistance, and heat resistance can be obtained, and at the same time, shrinkage during curing can be controlled to be low, and a highly accurate modeled object can be obtained. In addition, as described above, the contents of low molecular weight components in the modeled objects obtained in Examples are low, and the strength, water resistance, and heat resistance of the modeled objects are high. Modeled objects having such good properties were not obtained from the compositions of the Comparative Examples.

Examples 46 to 51 and Comparative Examples 16 to 18 (Preparation and evaluation of active energy ray curable cosmetic composition for nails)

The active energy ray curable composition obtained in Table 2, the polymerizable photoinitiator (A) or the photopolymerization initiator (D) shown in Table 1, and other components were weighed according to the proportions shown in Table 6 (solid content conversion), and uniformly mixed at room temperature to prepare a nail cosmetic composition. The curability of the nail cosmetic composition, the adhesion to a nylon substrate, and the surface hardness and surface gloss of the obtained cured film were evaluated, and the results are shown in Table 6.

<Evaluation of Curability of Nail Cosmetics>

The active energy ray curable nail cosmetic compositions prepared in Examples and Comparative Examples were applied onto a nylon 6 test piece ("SHT-N6 (NC)" manufactured by Toray Plastics Seiko Co., Ltd.) using a separator so that the film thickness was 100 $\mu$m, thereafter, ultraviolet rays were irradiated using a UV LED lamp (Manufactured by Beauty Nailer, wavelength 405nm, 48W) exclusively used for gel nails, and after curing, the surface was wiped with tissue paper to produce a cured film. The time required for the cured film to become tacky when touched was divided into four stages and evaluated. The shorter the time required for the tack to disappear, the higher the curability.

◎: Tack disappeared in less than 1 minute.
○: Tack disappeared in 1 minute or more, but less than 3 minutes
△: Tack disappeared in 3 minutes or more, but less than 10 minutes
×: Tack did not disappear even more than 10 minutes.

<Evaluation of Adhesion (Nylon substrate)>

Each of the obtained photocurable nail cosmetic compositions of Examples and Comparative Examples was applied onto a nylon 6 test piece in the same manner as described above, and irradiated with light for 3 minutes to prepare a cured film. On the surface of the obtained cured films 100 squares with 1 mm$^2$ were cross cut with a cutter knife in accordance with JIS K 5600, and the number of squares remaining on the test piece when a commercially available cellophane tape was bonded and then peeled off was evaluated in four grades, and the results are shown in Table 6. The greater the number of squares remaining on the test piece, the higher the adhesion.

◎: The number of remaining squares was 100.
○: The number of remaining squares was 90 to 99.
△: The number of remaining squares was 60 to 89.
×: The number of remaining squares was less than 60.

<Surface Hardness Evaluation of Cured Film>

Using the cured films of the respective Examples and Comparative Examples obtained in the adhesion evaluation, the surface of the film was pulled with a pencil having a hardness of HB by pressing a load of 750 g at an angle of 45 °, and the occurrence of peeling and the presence of scratches were visually confirmed and evaluated in three stages, and the results are shown in Table 6. The smaller the occurrence of scratches and peeling, the higher the surface hardness.

○: No scratches or peeling occurred. Surface hardness was greater than pencil hardness HB.
△: Peeling did not occur, but scratches occurred.
×: Peeling occurred.

<Surface Gloss Evaluation>

Using the cured films of Examples and Comparative Examples obtained in the adhesion evaluation, the gloss of the surface of the films was visually observed and evaluated according to the following criteria.

○: The gloss was confirmed.
△: Although reflection of light could be confirmed, cloudiness was observed.
×: Reflection of light could not be confirmed, and there was no gloss.

[Table 6]

| | | Nail Cosmetic Composition | | | | Curability | Adhesion | Surface Hardness | Surface gloss |
|---|---|---|---|---|---|---|---|---|---|
| | | Curable composition or photoinitiator | | Others | | | | | |
| | | Type | Mass (%) | Type | Mass (%) | | | | |
| Examples | 46 | Example 8 | 100 | - | - | ◎ | ◎ | ○ | ○ |
| | 47 | Example 2 | 80 | b3-6 | 13 | ○ | ◎ | ○ | ○ |
| | | Example 6 | 5 | C-2 | 2 | | | | |
| | 48 | Example 24 | 10 | b2-6 | 40 | ○ | ◎ | ○ | ○ |
| | | | | b3-5 | 50 | | | | |
| | 49 | Example 17 | 70 | b2-2 | 30 | ○ | ◎ | ○ | ○ |
| | 50 | A-6 | 8 | b1-10 | 20 | ◎ | ○ | ○ | ○ |
| | | A-14 | 2 | b2-4 | 20 | | | | |
| | | | | b3-2 | 50 | | | | |
| | 51 | A-17 | 20 | b1-6 | 25 | ◎ | ◎ | ◎ | ◎ |
| | | | | b2-2 | 30 | | | | |
| | | | | b3-4 | 25 | | | | |
| Comparative Examples | 16 | D-2 | 5 | b2-1 | 40 | × | △ | △ | × |
| | | | | b3-2 | 55 | | | | |
| | 17 | Comparative Example 5 | 20 | b2-1 | 25 | × | ○ | △ | × |
| | | | | b3-1 | 55 | | | | |
| | 18 | Comparative Example 6 | 50 | b3-2 | 50 | × | × | × | × |

As is clear from the results in Table 6, the active energy ray curable nail cosmetic compositions of the Examples had excellent curability with respect to a commercially available UV lamp dedicated to gel nails, and also had high adhesion to a nylon substrate (a material having a large number of amide bonds similar to nails which are a protein main component). From these results, it is understood that the active energy ray curable nail cosmetic composition of the present invention can be suitably used as a gel nail for a base gel to be directly applied to a nail. In addition, since the content of the low molecular weight component in the obtained cured film is low, the surface hardness and surface gloss of the cured film are good, and the cured film can be suitably used as a gel nail for top coating. On the other hand, since the active energy ray curable nail cosmetic compositions of Comparative Examples had low curability and contained a large amount of low molecular weight components in the cured films, the surface hardness and surface gloss of the cured films were low.

Examples 52 to 59 and Comparative Examples 19 to 21 (Preparation and evaluation of active energy ray curable pressure-sensitive adhesive compositions)

The active energy ray curable composition obtained in Table 2, the polymerizable photoinitiator (A) or the photopolymerization initiator (D) shown in Table 1, and other components were weighed according to the proportions shown in Table 7 (solid content conversion), and uniformly mixed at room temperature to prepare pressure-sensitive adhesive compositions. A pressure-sensitive adhesive sheet was prepared using the pressure-sensitive adhesive composition by the following method, and the curability of the pressure-sensitive adhesive composition, the adhesion to various substrates, and the transparency, adhesive strength, stain resistance (reworkability), durability and light yellowing resistance of the obtained adhesive sheet were evaluated, and the results are shown in Table 7.

<Curability Evaluation of Adhesive>

A 75 $\mu$m-thick heavy-release PET film (manufactured by Toyobo Co., Ltd., polyester film E7001) was brought into close contact with a horizontally placed glass plate, a spacer with the thickness of 1 mm and an internal size of 60 mm $\times$ 100 mm was placed, the prepared active energy ray curable pressure-sensitive adhesive composition of Examples and Comparative Examples was filled inside the spacer, a 50 $\mu$m-thick light-release PET film (manufactured by Toyobo Co., Ltd., polyester film E7002) was further placed thereon, and irradiation was performed by a UVLED lamp having a wave length 385 nm and a power 100 mW/cm$^2$ so that an cumulative amount of light is 3000 mJ/cm$^2$ to cure the adhesive composition. Thereafter, the release PET films on both sides were removed, and the obtained cured product was touched to evaluate the curability in three stages.

O: A cured product that maintains its shape was obtained, and although tack was observed when the cured product was touched, there was no adhesion of liquid uncured material.
△: A cured product that can maintain its shape was obtained, tack was observed when the cured product was touched, and liquid uncured material was attached.
×: Curing is insufficient, a cured product that retains its shape could not be obtained, and a large amount of liquid residue was observed.

<Preparation of Pressure-sensitive Adhesive Sheet and Adhesion Evaluation>

The active energy ray curable pressure-sensitive adhesive composition prepared above was applied onto various plate-shaped or film-shaped substrates (substrates) shown below, and the pressure-sensitive adhesive layer was laminated to a thickness of 50 $\mu$m using a table-top roll laminator (RSL-382S, manufactured by Royal Sovereign) to avoid air bubbles with a light-release separator (Silicone coated PET film), and then irradiated with ultraviolet rays (UVLED lamp: wavelength 385nm, illuminance 1000mW/cm$^2$, cumulative light amount 2000 mJ/cm$^2$). Thereafter, the light-release separator was peeled off to obtain a pressure-sensitive adhesive sheet composed of the pressure-sensitive adhesive layer and the substrate. Using the obtained pressure-sensitive adhesive sheet, 100 squares with 1 mm$^2$ were cross-cut with a cutter knife in accordance with JIS K 5600, a cellophane tape was attached, and the number of squares in which the pressure-sensitive adhesive layer remained on the substrate side when the tape was peeled off at once was counted, and the adhesion was evaluated according to the following criteria.

Base materials (substrates)

PET: E5100 (corona-treated surface) manufactured by Toyobo Co. Ltd
PMMA: Comoglass P manufactured by Kuraray Co. Ltd
PC: PC1600 manufactured by C.I. Takiron
PVC: Esviron plate I-500 manufactured by Sekisui Plastics Co. Ltd
Glass (GL): Eagle XG manufactured by Corning Co. Ltd

Evaluation criteria

◎: 100 pieces without peeling
○: 95 to 99 pieces without peeling

△: 70 to 94 pieces without peeling
×: 0 to 69 pieces without peeling

<Adhesive Strength Evaluation>

A pressure-sensitive adhesive sheet was prepared in the same manner as described above, and the pressure-sensitive adhesive layer was transferred to an easily adhesive surface of an easily adhesive PET film (Cosmoshine A4160, manufactured by Toyobo Co., Ltd) under conditions of a temperature of 23°C and a relative atmospheric humidity of 50%, and the film was pressure-bonded by moving back and forth twice using a pressure-bonding roller having a weight of 2 kg, and allowed to stand in the same atmosphere for 30 minutes. Thereafter, the 180° peel strength (N/25 mm) was measured at a peel rate of 300 mm/min according to JIS Z0237 using a tensile tester (apparatus name: TENSILON RTA-100, manufactured by ORIENTEC Co., Ltd), and adhesive strength was evaluated according to the following criteria.

◎: 20 (N/25mm) or more
○: 10 (N/25mm) or more and less than 20 (N/25mm)
△: 5 (N/25mm) or more and less than 10 (N/25mm)
×: less than 5 (N/25mm)

<Transparency Evaluation of Adhesive Sheet>

The total light transmittance of the glass substrate was measured in accordance with JIS K 7105 using a haze meter (NDH 2000, manufactured by Nippon Denshoku Industries Co., Ltd). A pressure-sensitive adhesive sheet on a glass substrate was prepared in the same manner as described above, the pressure-sensitive adhesive layer was transferred to the glass substrate under conditions of a temperature of 23°C and a relative humidity of 50%, and the total light transmittance of the glass substrate and the pressure-sensitive adhesive layer was measured. Then, the transmittance of the adhesive layer itself was calculated by subtracting the transmittance of the glass substrate, and the transparency was evaluated in four grades as follows.

◎: Transmission was 90% or more.
○: transmission was 85% or more and less than 90%.
△: Transmission was 50% or more and less than 85%.
×: Transmission was less than 50%.

<Stain Resistance (Reworkability) Evaluation>

A pressure-sensitive adhesive sheet was prepared in the same manner as in the measurement of the pressure-sensitive adhesive strength described above, allowed to stand at 80°C for 24 hours, and then the pressure-sensitive adhesive sheet was peeled off, and contamination (remaining state of the pressure-sensitive adhesive layer (glue)) on the surface of the substrate film was visually observed and evaluated according to the following criteria.

◎: There was no contamination (no glue residue).
○: There was very little contamination.
△: There was slight contamination.
×: There was contamination (with glue residue).

<Evaluation of Light Yellow Discoloration Resistance>

A pressure-sensitive adhesive sheet on a glass substrate was prepared in the same manner as above, set in a xenon fade meter (SC-700-WA: Manufactured by Suga Test Instruments Co., Ltd.), and irradiated with ultraviolet rays with an intensity of 70 mW/cm$^2$ for 120 hours, and then visually observed for discoloration of the pressure-sensitive adhesive layer on the sheet. Evaluation was made according to the following criteria.

◎: Yellowing could not be visually observed at all.
○: Very slight yellowing could be visually observed.
△: Slight yellowing could be visually observed.
×: A clear yellowing could be visually observed.

<Durability Evaluation>

A pressure-sensitive adhesive sheet on a glass substrate was prepared in the same manner as above, and after standing for 100 hours under conditions of a temperature of 85°C and a relative humidity of 85%, the presence or absence of lifting or peeling of the pressure-sensitive adhesive layer, bubbles and cloudiness was visually observed and evaluated according to the following criteria.

◎: It was transparent, and neither lifting nor peeling nor bubbles was observed.
×: It was clouding, lifting or flaking, and bubbles was observed.

[Table 7]

| Pressure-sensitive adhesive composition | | | | Curability | Trans parency | Adhesion | | | | | Adhesive strength | | | | Stain resistance | | | Light yellow discoloration resistance | Durability |
|---|---|---|---|---|---|---|---|---|---|---|---|---|---|---|---|---|---|---|---|
| Curable composition or photoinitiator | | Others | | | | PET | PMMA | PC | PVC | GL | PET | PMMA | PC | GL | PET | PC | GL | | |
| Type | Mass (%) | Type | Mass (%) | | | | | | | | | | | | | | | | |

| | | Pressure-sensitive adhesive composition | | | | Curability | Transparency | Adhesion | | | | | Adhesive strength | | | | Stain resistance | | | Light yellow discoloration resistance | Durability |
|---|---|---|---|---|---|---|---|---|---|---|---|---|---|---|---|---|---|---|---|---|---|
| | | Curable composition or photoinitiator | | Others | | | | PET | PMMA | PC | PVC | GL | PET | PMMA | PC | GL | PET | PC | GL | | |
| | | Type | Mass (%) | Type | Mass (%) | | | | | | | | | | | | | | | | |
| Examples | 52 | Example 5 | 50 | b2-4 | 15 | ○ | ◎ | ◎ | ○ | ◎ | ○ | ○ | ◎ | ○ | ◎ | ○ | ◎ | ◎ | ◎ | ◎ | ◎ |
| | | | | b3-4 | 35 | | | | | | | | | | | | | | | | |
| | 53 | Example 7 | 20 | b1-1 | 5 | ○ | ◎ | ◎ | ○ | ◎ | ○ | ◎ | ○ | ○ | ◎ | ◎ | ◎ | ◎ | ◎ | ◎ | ◎ |
| | | | | b2-2 | 35 | | | | | | | | | | | | | | | | |
| | | | | b3-6 | 40 | | | | | | | | | | | | | | | | |
| | 54 | Example 9 | 10 | b3-10 | 10 | ○ | ◎ | ◎ | ○ | ◎ | ○ | ○ | ◎ | ○ | ◎ | ○ | ◎ | ◎ | ◎ | ◎ | ◎ |
| | | Example 20 | 80 | | | | | | | | | | | | | | | | | | |
| | 55 | Example 21 | 10 | b1-7 | 20 | ○ | ◎ | ◎ | ◎ | ◎ | ◎ | ◎ | ◎ | ◎ | ◎ | ◎ | ◎ | ◎ | ◎ | ○ | ◎ |
| | | | | b3-3 | 40 | | | | | | | | | | | | | | | | |
| | | A-7 | 5 | b2-1 | 15 | | | | | | | | | | | | | | | | |
| | | | | b2-2 | 10 | | | | | | | | | | | | | | | | |
| | 56 | Example 15 | 40 | b 1-3 | 20 | ○ | ◎ | ◎ | ○ | ◎ | ○ | ◎ | ◎ | ○ | ◎ | ◎ | ◎ | ◎ | ◎ | ◎ | ◎ |
| | | A-16 | 5 | b2-1 | 20 | | | | | | | | | | | | | | | | |
| | | | | b2-4 | 15 | | | | | | | | | | | | | | | | |
| | 57 | A-17 | 5 | b2-4 | 40 | ○ | ◎ | ◎ | ○ | ◎ | ○ | ◎ | ◎ | ○ | ○ | ◎ | ◎ | ◎ | ◎ | ○ | ◎ |
| | | | | b3-3 | 55 | | | | | | | | | | | | | | | | |
| | 53 | A-8 | 1 | b1-2 | 20 | ○ | ○ | ◎ | ○ | ○ | ○ | ◎ | ◎ | ○ | ○ | ◎ | ○ | ○ | ◎ | ○ | ◎ |
| | | | | b2-1 | 30 | | | | | | | | | | | | | | | | |
| | | | | b2-4 | 9 | | | | | | | | | | | | | | | | |
| | | | | b3-3 | 40 | | | | | | | | | | | | | | | | |
| | 59 | A-6 | 10 | b1-8 | 50 | ○ | ◎ | ◎ | ◎ | ◎ | ◎ | ○ | ◎ | ◎ | ◎ | ○ | ◎ | ◎ | ◎ | ◎ | ◎ |
| | | A-18 | 2 | b2-2 | 38 | | | | | | | | | | | | | | | | |

(continued)

| Comparative Examples | | Pressure-sensitive adhesive composition | | | | Curability | Trans parency | Adhesion | | | | | Adhesive strength | | | | Stain resistance | | | Light yellow discoloration resistance | Durability |
|---|---|---|---|---|---|---|---|---|---|---|---|---|---|---|---|---|---|---|---|---|---|
| | | Curable composition or photoinitiator | | Others | | | | PET | PMMA | PC | PVC | GL | PET | PMMA | PC | GL | PET | PC | GL | | |
| | | Type | Mass (%) | Type | Mass (%) | | | | | | | | | | | | | | | | |
| 19 | | Comparative Example 2 | 70 | b3-3 | 20 | Δ | ○ | Δ | × | Δ | × | × | Δ | × | Δ | × | × | × | × | Δ | × |
| | | | | b3-10 | 10 | | | | | | | | | | | | | | | | |
| 20 | | Comparative Example 5 | 40 | b3-1 | 40 | Δ | Δ | × | × | Δ | × | × | × | × | Δ | × | × | × | × | × | × |
| | | | | b2-1 | 15 | | | | | | | | | | | | | | | | |
| 21 | | D-2 | 5 | b1-10 | 45 | × | × | Δ | × | × | × | × | Δ | × | × | × | × | × | × | × | × |
| | | D-4 | 30 | C3-10 | 25 | | | | | | | | | | | | | | | | |

39

As is clear from the results in Table 7, the compositions for active energy ray curable pressure-sensitive adhesives of Examples had high curability, and the pressure-sensitive adhesive sheets obtained by curing the compositions had high transparency and good adhesiveness and pressure-sensitive adhesiveness (pressure-sensitive adhesive force) to various materials. In particular, the cured product (pressure-sensitive adhesive sheet) obtained from the active energy ray curable pressure-sensitive adhesive composition of the present invention had a low content of low molecular weight components, so that the cured product was excellent in adhesive residue resistance when peeled from a substrate, and also excellent in yellowing resistance and durability. On the other hand, in the compositions of Comparative Examples, since the curability was low and the curing did not proceed sufficiently, both the adhesion to the material and the pressure-sensitive adhesiveness were low, and since the cured product contained a large amount of low molecular weight components such as remaining uncured components or decomposition products generated by ultraviolet irradiation, it was found that the stain resistance, the yellowing resistance and the durability of the cured product were all low.

Examples 60 to 64 and Comparative Examples 22 to 24 (Preparation and evaluation of active energy ray curable adhesive compositions)

The active energy ray curable composition obtained in Table 2, the polymerizable photoinitiator (A) or the photopolymerization initiator (D) shown in Table 1, and other components were weighed according to the proportions shown in Table 8 (solid content conversion), and uniformly mixed at room temperature to prepare an adhesive composition. Using the adhesive composition, a laminated film bonded by the following method was produced, and the curability of the adhesive composition, the adhesive strength to various substrates, and the durability of the obtained laminated film were evaluated, and the results are shown in Table 8.

<Curability Evaluation of Adhesive Composition>

A PET film (manufactured by Toyobo Co., Ltd., polyester film E5100) was brought into close contact with a horizontally placed glass plate with the corona-treated side facing the surface. The active energy ray curable adhesive compositions of Examples and Comparative Examples were applied using a bar coater No. 12 so as to have a film thickness of 20 $\mu$m, and overlaid with a 50 $\mu$m-thick light-release PET film (Polyester Film E7002, manufactured by Toyobo Co., Ltd), and irradiated with ultraviolet rays using a UVLED lamp with wavelengths 365 nm and power 50 mW/cm$^2$, the adhesive composition was cured. Thereafter, the light-release PET film was removed, the presence or absence of tack on the surface of the cured film was confirmed, and the curability was evaluated according to the following criteria based on the cumulative amount of light required until the tack disappeared.

◎: Tack disappeared when the cumulative amount of light was less than 1000 mJ/cm$^2$.
○: Tack disappeared when the cumulative light amount was 1000 mJ/cm$^2$ or more and less than 2000 mJ/cm$^2$.
△: Tack disappeared when the cumulative light amount was 2000 mJ/cm$^2$ or more and less than 5000 mJ/cm$^2$.
×: Tack remained even when the cumulative amount of light was 5000 mJ/cm$^2$ or more.

<Production of Laminated Film and Adhesive Strength Evaluation>

After the active energy ray curable adhesive composition prepared above was coated on various plate-shaped or film-shaped base materials (substrates) shown below, E5100 manufactured by Toyobo Co., Ltd. (corona treated surface) was laminated on it using a table-top roll laminator (RSL-382S manufactured by Royal Sovereign) to avoid air bubbles to obtain an adhesive layer with a thickness of 20 $\mu$m. Then the obtained adhesive layer was irradiated by ultraviolet rays (UVLED lamp with a wavelength of 365 nm, power 50 mW/cm$^2$, cumulative light amount: 5000 mJ/cm$^2$) to produce a laminated film. Thereafter, the 180° peel strength (N/25 mm) was measured at a peel rate of 300 mm/min according to JIS Z0237 using a tensile tester (apparatus name: Tensilon RTA-100 manufactured by ORIENTEC Co., Ltd), and the adhesive strength was evaluated according to the following criteria.

Base material (substrate)

PET (not yet): E5100 (untreated surface) manufactured by Toyobo Co. Ltd
PMMA: Comoglass P manufactured by Kuraray Co. Ltd
PC: PC1600 manufactured by C.I. Takiron

Evaluation criteria

◎: 20 (N/25mm) or more
○: 10 (N/25mm) or more and less than 20 (N/25mm)
△: 5 (N/25mm) or more and less than 10 (N/25mm)
✕: less than 5 (N/25mm)

<Durability Evaluation of Laminated Film>

Similar to the adhesive strength evaluation described above, laminated films of E5100 manufactured by Toyobo Co., Ltd. (corona treated surface) and various film-shaped substrates such as PET film were prepared, after being maintained at a temperature of 85°C and a relative humidity of 85% for 100 hours, the adhesive layer was visually observed for lifting, peeling, bubbles, and cloudiness, and the durability was evaluated according to the following criteria.

◎: It was transparent no lifting, peeling, or bubbles.
○: It had a very slight cloudiness, but no lifting, peeling, or bubbles.
△: It had a slight cloudiness, or had slight lifting, slight peeling, or slight bubbles.
✕: It was extremely cloudy or floating, flaking, and bubbles.

[Table 8]

| | | | Adhesive composition | | | | Curability | Adhesion | | | Durability |
|---|---|---|---|---|---|---|---|---|---|---|---|
| | | | Curable composition or photoinitiator | | Others | | | PET (untreated surface) | PMMA | PC | |
| | | | Type | Mass | Type | Mass | | | | | |
| Examples | | 60 | Example 1 | 100 | - | - | ○ | ○ | ◎ | ○ | ○ |
| | | 61 | Example 3 | 20 | b2-1 | 30 | ○ | ○ | ○ | ○ | ◎ |
| | | | A-11 | 10 | b3-7 | 40 | | | | | |
| | | 62 | Example 5 | 60 | b3-6 | 20 | ◎ | ◎ | ◎ | ○ | ◎ |
| | | | | | C-1 | 20 | | | | | |
| | | 63 | Example 13 | 40 | b1-10 | 5 | ◎ | ○ | ○ | ◎ | ◎ |
| | | | | | b3-5 | 20 | | | | | |
| | | | Example 14 | 20 | b2-1 | 10 | | | | | |
| | | | | | C-6 | 5 | | | | | |
| | | 64 | Example 16 | 50 | b1-4 | 10 | ○ | ○ | ○ | ○ | ○ |
| | | | | | b3-8 | 30 | | | | | |
| | | | | | b3-10 | 10 | | | | | |
| Comparative Examples | | 22 | Comparative Example 5 | 40 | b2-1 | 10 | △ | △ | ✕ | △ | △ |
| | | | | | b3-1 | 30 | | | | | |
| | | | | | b3-6 | 20 | | | | | |
| | | 23 | Comparative Example 6 | 50 | b3-3 | 30 | ✕ | ✕ | ✕ | ✕ | ✕ |
| | | | | | b3-8 | 20 | | | | | |
| | | 24 | D-2 | 5 | b3-3 | 70 | ✕ | ✕ | ✕ | ✕ | ✕ |
| | | | | | b3-5 | 10 | | | | | |
| | | | | | b3-11 | 15 | | | | | |

As is clear from the results in Table 8, the compositions for active energy ray curable adhesives of Examples had high curability, and the laminated films obtained by curing the compositions had high adhesion to various materials and were good. In particular, a cured product (adhesive layer of a laminated film) obtained from the active energy ray curable adhesive composition of the present invention has a low content of a low molecular weight component, and thus has good durability and exhibits characteristics suitable for an adhesive or a sealing material. On the other hand, since the compositions of Comparative Examples had low curability and were not sufficiently cured, the adhesive strength to the substrate was low, and a large amount of low molecular weight components such as uncured components or decomposition products generated by ultraviolet irradiation remained in the cured product, so that the durability of the adhesive layer was also low.

Examples 65 to 67 and Comparative Examples 25 and 26 (Preparation and evaluation of active energy ray curable coating agent compositions)

The active energy ray curable composition obtained in Table 2, the polymerizable photoinitiator (A) or the photopolymerization initiator (D) shown in Table 1, and other components were weighed according to the proportions shown in Table 9 (solid content conversion), and uniformly mixed at room temperature to prepare a coating agent composition. Using the coating agent composition, a coating layer was produced by the following method, and the curability of the coating agent composition and the adhesion, bending resistance, chemical resistance, scratch resistance, self-repairing property, and durability of the obtained coating layer were evaluated, and the results are shown in Table 9.

<Curability Evaluation of Coating Agent Composition>

A PET film (manufactured by Toyobo Co., Ltd., polyester film E5100) was brought into close contact with a horizontally placed glass plate with the corona-treated side facing the surface. The active energy ray curable coating agent compositions of Examples and Comparative Examples were applied using a bar coater No. 6 so as to have a film thickness of 10 $\mu$m, and irradiated with ultraviolet rays using a metal halide lamp (apparatus: inverter-type conveyor apparatus ECS-4011GX manufactured by Eye Graphics Co., Ltd., metal halide lamp: M04-L41 manufactured by Eye Graphics Co., Ltd., ultraviolet illuminance 500 mW/cm$^2$) under nitrogen atmosphere, the coating composition was cured. Thereafter, the presence or absence of tack on the surface of the cured film was confirmed, and the curability was evaluated according to the following criteria based on the cumulative amount of light required until the tack disappeared.

◎: Tack disappeared when the cumulative amount of light was less than 1000 mJ/cm2.
○: Tack disappeared when the cumulative light amount was 1000 mJ/cm2 or more and less than 2000 mJ/cm2.
△: Tack disappeared when the cumulative light amount was 2000 mJ/cm2 or more and less than 5000 mJ/cm 2.
✕: Tack remained even when the cumulative amount of light was 5000 mJ/cm2 or more.

<Preparation of Coating Layer and Adhesion Evaluation>

A PET film (manufactured by Toyobo Co., Ltd., polyester film E5100) was brought into close contact with a horizontally placed glass plate with the corona-treated side facing the surface. The active energy ray curable coating agent compositions of Examples and Comparative Examples were applied using a bar coater No. 12 so as to have a film thickness of 20 $\mu$m, and irradiated with ultraviolet rays using a UVLED lamp with wavelengths 365 nm at an illumination intensity of 500 mW/cm$^2$ and a cumulative amount of light of 5000 mJ/cm$^2$, and a coating layer was formed on the PET film. Using the obtained coating layer, 100 squares with 1 mm$^2$ were cross-cut with a cutter knife in accordance with JIS K 5600, a cellophane tape was attached, and the number of squares in which the pressure-sensitive adhesive layer remained on the substrate side when the tape was peeled off at once was counted, and the adhesion was evaluated according to the following criteria.

◎: 100 pieces without peeling
○: 95 to 99 pieces without peeling
△: 70 to 94 pieces without peeling
✕: 0 to 69 pieces without peeling

<Bending Resistance Evaluation of Coating Layer>

A laminate composed of a PET film and a coating layer prepared in the same manner as in the adhesion evaluation described above, according to the cylindrical mandrel method described in JIS K-5600, the laminate was brought into contact with a mandrel (10 mm φ) so that the coat layer was on the outside, and after being bent, the presence or absence of cracks in the coat layer was visually observed, and the bending resistance was evaluated according to the following criteria.

◎: No cracking was observed.
○: Some of the bent parts turned white.
△: Some cracks were observed at the bent portion.
×: Cracks were observed at the bent portion.

<Chemical Resistance Evaluation of Coating Layer>

A coat layer was prepared on the PET film in the same manner as in the adhesion evaluation described above, and oleic acid was applied to the surface of the coat layer to a diameter of about 1 cm, the coating layer was kept at 23°C for 1 hour, washed off with a neutral detergent, the surface state was observed, and chemical resistance was evaluated according to the following criteria.

◎: No trace of oleic acid was observed at all.

A very thin trace of whitening was seen in the area where oleic acid was applied.

△: The area to which oleic acid was applied turned white and surface swelling was observed.
×: The area coated with oleic acid was sticky and surface peeling was observed.

<Scratch Resistance Evaluation of Coat Layer>

A coating layer was prepared on a PET film in the same manner as in the adhesion evaluation described above, and the surface of the coating layer was rubbed 10 times with #0000 steel wool at a load of 100 g at a room temperature of 23°C and a humidity of 50%, thereafter the surface of the coating layer was visually observed, and the scratch resistance was evaluated according to the following criteria.

◎: No peeling of the coating layer or occurrence of scratches was observed.
○: A few thin scratches were observed in a part of the coating layer.
△: Streak scratches were observed throughout the coating layer.
×: Peeling of the coating layer was observed.

<Self-Repairing Property Evaluation of Coating Layer>

A coating layer was prepared on a PET film in the same manner as in the adhesion evaluation described above, and the surface of the coating layer was rubbed 10 times with a brass brush at a load of 100 g at a room temperature of 23°C and a humidity of 50%, thereafter the surface of the coating layer was visually observed, and the self-Repairing Property was evaluated according to the following criteria.

◎: Scratches were restored within 30 minutes. Or no scratch was observed.
○: Scratches were observed after 30 minutes, but were restored after 24 hours or by holding at 60°C for 8 hours.
×: Scratches were observed after 24 hours, and the scratches were not restored even after being held at 60°C for 8 hours.

<Durability Evaluation of Coating Layer>

A coating layer was prepared on a PET film in the same manner as in the adhesion evaluation described above, and after holding for 100 hours under conditions of a temperature of 85°C and a relative humidity of 85%, the presence or absence of lifting or peeling of the coating layer, generation of bubbles or cloudiness was visually observed, and the durability was evaluated according to the following criteria.

◎: It was transparent no lifting, peeling, or bubbles.
○: It had a very slight cloudiness, but no lifting, peeling, or bubbles.
×: It was extremely cloudy or floating, flaking, and bubbles.

[Table 9]

| | | Coating composition | | | | Curability | Adhesion | Bending resistance | Chemical resistance | Scratch resistance | Self-repairing property | Durability |
|---|---|---|---|---|---|---|---|---|---|---|---|---|
| | | Curable composition or initiator | | Others | | | | | | | | |
| | | Type | Mass (%) | Type | Mass (%) | | | | | | | |
| Examplse | 65 | Example 7 | 40 | b2-7 | 25 | ◎ | ○ | ◎ | ○ | ○ | ◎ | ◎ |
| | | Example 10 | 30 | C-6 | 5 | | | | | | | |
| | 66 | Example 21 | 15 | b1-2 | 35 | ○ | ◎ | ◎ | ○ | ◎ | ○ | ○ |
| | | | | b2-9 | 25 | | | | | | | |
| | | | | b3-11 | 25 | | | | | | | |
| | 67 | A-11 | 20 | b1-1 | 30 | ◎ | ◎ | ○ | ◎ | ◎ | ○ | ◎ |
| | | | | b2-5 | 20 | | | | | | | |
| | | | | b3-2 | 30 | | | | | | | |
| Comparative Examples | 25 | Comparative Example 7 | 50 | b3-5 | 20 | Δ | Δ | × | × | Δ | × | × |
| | | | | b2-9 | 30 | | | | | | | |
| | 26 | D-1 | 20 | b2-5 | 20 | Δ | Δ | Δ | × | × | × | × |
| | | | | b3-2 | 30 | | | | | | | |
| | | | | b3-11 | 30 | | | | | | | |

As is clear from the results in Table 9, the compositions for active energy ray curable coating agents of Examples have high curability, and the coating layers obtained by curing the compositions exhibit good adhesion, and also have bending resistance and scratch resistance. In particular, the cured product (coating layer) obtained from the active energy ray curable coating agent composition of the present invention has a low content of low molecular weight components, and therefore has good durability, and exhibits excellent properties for coating agents for vehicles and indoor and outdoor applications. Further, it exhibited a self-repairing property and was expected to be applied to a self-repairing paint, and it was also excellent in chemical resistance, so that it could be used as a coating layer for decoration. On the other hand, since the compositions of Comparative Examples had low curability and were not sufficiently cured, the adhesion to the substrate was low, the bending resistance and scratch resistance were insufficient, and the self-repairing property was not exhibited. Furthermore, since a large amount of low molecular weight components such as uncured components or decomposition products generated by ultraviolet irradiation remained in the cured product, the durability of the coating layer was also low.

Examples 68 to 70 and Comparative Examples 27 and 28 (Preparation and evaluation of active energy ray curable dental compositions)

The active energy ray curable composition obtained in Table 2, the polymerizable photoinitiator (A) or the photopolymerization initiator (D) shown in Table 1, and other components were weighed according to the proportions shown in Table 10 (solid content conversion), and uniformly mixed at room temperature to prepare an active energy ray curable dental composition. The solubility or dispersibility (when an insoluble inorganic filler, pigment, or the like is blended) of the dental composition was visually observed to evaluate the storage stability, and the results are shown in Table 10. Further, using the dental composition, a dental material was prepared by the following method, and the curability of the dental composition and the surface smoothness, hardness and adhesive strength of the obtained dental material were evaluated, and the results are shown in Table 10.

<Solubility (Dispersibility)>

◎: The obtained composition was homogeneous and transparent.
○: The obtained composition was homogeneous and translucent.
△: The obtained composition was turbid and the uniformity was difficult to judge.
×: The obtained composition was not completely miscible.

<Storage Stability>

Each of the active energy ray curable dental compositions obtained in Examples 68 to 70 and the active energy ray curable compositions obtained in Comparative Examples 27 and 28 was placed in a light-shielding screw tube, the cap was closed, and the tube was stored under two conditions of 40°C for 1 month and 80°C for 2 weeks. The dissolved or dispersed state of the composition after storage was confirmed to evaluate storage stability.

◎: There was no change in state after storage for both conditions at 40°C for 1 month and at 80°C for 2 weeks.
O: Changes in state after storage were confirmed at either one condition at 40°C for 1 month or at 80°C for 2 weeks.
×: Changes in state after storage were confirmed under both conditions at 40°C for 1 month and at 80°C for 2 weeks.

<Curability>

Each of the active energy ray curable dental compositions obtained in Examples 68 to 70 and the active energy ray curable compositions obtained in Comparative Examples 27 and 28 was filled in a polytetrafluoroethylene mold (20 mm × 20 mm × 10 mm) having a hole of 6 mm diameter at the center, pressed with a polypropylene film, and a dental light irradiator (Tokuso Powerlight, manufactured by Tokuyama Dental Co., Ltd., optical power density: 700 mW/cm$^2$, light intensity on irradiated surface: 640 to 650 mW/cm$^2$, light sources: halogen lamps, irradiation aperture: 8 mm) was brought into close contact with the polypropylene film to irradiate it for 30 seconds. The polypropylene film was peeled off and the cured product was touched with the hand to check for stickiness and the presence of uncured components.

◎: There was no stickiness (completely cured).
○: There was some stickiness, but no finger marks remain on the surface (almost completely cured, no need to wipe off uncured components).

△: There were stickiness and finger marks remain on the surface (incompletely cured, uncured components need to be wiped off).

×: There were severe stickiness and fingers stick to surfaces (many uncured components remain and cannot be used as a cured film).

<Surface Smoothness>

The surface of the cured product obtained in the curability evaluation was visually observed to confirm smoothness and glossiness.

◎: The surface was smooth and shiny.
○: The surface was almost smooth, and slightly cloudy or slight irregularities was observed.
△: The surface was entirely cloudy, and some irregularities or some grains was observed. ×: The surface was totally cloudy and covered with grains.

<Hardness>

The surface of the cured product obtained in the curability evaluation was buffed, and the Knoop hardness was measured using a microhardness meter manufactured by Matsuzawa Seiki under a load of 10 g for 20 seconds. Note that the measurement temperature was 23°C.

◎: Knoop hardness was 200 KHN or more (equivalent to permanent tooth enamel).
○: Knoop hardness was 70 KHN or more and less than 200 KHN (equivalent to dentin).
△: Knoop hardness was less than 70 KHN.
×: Measurement could not be performed because it was not cured.

<Adhesive Strength (Dentin Adhesive Strength)>

The bovine lower forehead front teeth were polished with #1000 water-resistant abrasive paper under water injection, a flat dentine surface for bonding was cut out, dried by blowing compressed air for 10 seconds, and a tape having a hole with a diametral 3 mm was attached to set an adhesive surface. Thereafter, an adhesive test piece was prepared by a known method (see the method described in Japanese Patent Publication 2010-208964). The adhesion test piece was immersed in water at 37°C for 24 hours, the tensile adhesive strength was measured with an Instron universal tester (cross head speed 2 mm/min), and was defined as the adhesive strength of the active energy ray curable dental compositions obtained in Examples 1 to 10 and the active energy ray curable compositions obtained in Comparative Examples 1 to 3 to enamels and dentins. The value of the tensile adhesive strength is an average value of five test pieces.

◎: Both enamel adhesive strength and dentin adhesive strength were 20 Mpa or more.
○: Only one of the adhesive strength of enamel or dentin was 20 Mpa or more.
△: Both enamel adhesive strength and dentin adhesive strength were 7 MPa or more.
×: Both enamel adhesive strength and dentin adhesive strength were less than 7 MPa.

[Table 10]

| | | Dental composition | | | | Solubility or dispersiveness | Curability | Hardness | Surface Smoothness | Adhesion strength | Storage stability |
|---|---|---|---|---|---|---|---|---|---|---|---|
| | | Curable composition or initiator | | Others | | | | | | | |
| | | Type | Mass (%) | Type | Mass (%) | | | | | | |
| Examples | 68 | Example 10 | 20 | b1-6 | 20 | ◎ | ○ | ◎ | ○ | ○ | ◎ |
| | | Example 11 | 20 | b2-1 | 35 | | | | | | |
| | 69 | Example 14 | 40 | b1-1 | 10 | ◎ | ◎ | ◎ | ◎ | ○ | ○ |
| | | | | b2-2 | 20 | | | | | | |
| | | | | b2-7 | 20 | | | | | | |
| | | | | C-8 | 10 | | | | | | |
| | 70 | A-8 | 20 | b1-1 | 10 | ○ | ○ | ○ | ◎ | ◎ | ○ |
| | | | | b1-12 | 10 | | | | | | |
| | | | | b2-10 | 30 | | | | | | |
| | | | | b3-3 | 25 | | | | | | |
| | | | | C-9 | 5 | | | | | | |
| Comparative Examples | 27 | Comparative Example 7 | 30 | b3-5 | 40 | △ | × | × | × | △ | × |
| | | | | b2-9 | 30 | | | | | | |
| | 28 | D-2 | 5 | b1-3 | 35 | △ | △ | △ | × | × | × |
| | | | | b2-7 | 30 | | | | | | |
| | | | | b3-9 | 30 | | | | | | |

EP 4 424 719 A1

As is apparent from the results shown in Table 10, the active energy ray curable dental compositions of Examples have high solubility (or dispersibility), curability and storage stability, and the cured products obtained by curing the compositions exhibit good hardness and have surface smoothness and adhesive strength. In particular, the cured product obtained from the active energy ray curable dental composition of the present invention had a low content of low molecular weight components, and was excellent in safety. On the other hand, since the compositions of Comparative Examples were low in solubility, curability and storage stability and were not sufficiently cured, the obtained cured products were low in hardness and surface smoothness and were insufficient in adhesive strength. Further, since a large amount of low molecular weight components such as uncured components or decomposition products generated by ultraviolet irradiation remained in the cured product, the safety of the cured product was low.

As shown in the evaluation results of Examples and Comparative Examples, the active energy ray curable composition containing the polymerizable photoinitiator (A) having a specific structure and the polymerizable compound (B) according to the present invention exhibites good curability due to high initiation efficiencies of A and no byproducts of low molecular weight components, and can be completely cured even with long-wavelength rays from UV-LED lamps having wavelengths of 365 nm to 405 nm, which do not contain short-wavelength ultraviolet rays such as UV-B and UV-C. In addition, the polymerizable photoinitiator (A) and the polymerizable compound (B) have good compatibility with each other, and a highly transparent curable composition and a cured product applicable to the optical field can be obtained. It was apparent that the content of a component having a molecular weight of less than 1,000 in the obtained cured product can be reduced to less than 10%, and the cured product has low odor, high safety, and excellent water resistance and durability. On the other hand, a curable composition not containing the polymerizable photoinitiator (A) or the polymerizable compound (B) does not exhibit sufficient curability, and in particular, the curability with respect to long-wavelength light from a UV-LED lamp having wavelengths of 365 nm to 405 nm is low. In addition, a large amount of low molecular weight components remains in the obtained cured product, and it is apparent that the cured product is inferior in safety and durability due to problems such as odor generation, bleeding out and coloring with time. That is, it can be confirmed that the characteristics of the active energy ray curable composition of the present invention and the cured product obtained therefrom are due to the interaction between the polymerizable photoinitiator (A) and the polymerizable compound (B) contained therein.

INDUSTRIAL APPLICABILITY

As described above, the active energy ray curable composition of the present invention exhibits high curability, and can be cured using various light sources ranging from a metal halide lamp to a UVLED lamp having a wavelength of 405 nm. In addition, the amount of low molecular weight components in the cured product is small, and the molded product obtained by a method such as three-dimensional modeling has a low odor, high safety, and high durability. The active energy ray curable composition of the present invention can be suitably used as an active energy ray curable ink composition, an active energy ray curable inkjet ink composition, an active energy ray curable nail cosmetic composition, an active energy ray curable dental composition, an active energy ray curable pressure-sensitive adhesive composition, an active energy ray curable adhesive composition, an active energy ray curable sealing material composition, an active energy ray curable coating composition, an active energy ray curable composition for decorative sheets, an active energy ray curable composition for self-repairing materials, an active energy ray curable elastomer composition, an active energy ray curable composition for three-dimensional modeling, an active energy ray curable coating composition for vehicles, an active energy ray curable coating composition for buildings, and the like.

**Claims**

1. An active energy ray curable composition comprising:

   a polymerizable photoinitiator (A) having one or more benzophenone groups and one or more ethylenically unsaturated groups per one molecule and
   a polymerizable compound (B) (excluding A) having one or more ethylenically unsaturated groups per one molecule, wherein a content of components having a molecular weight of less than 1000 in a cured product of the active energy ray curable composition is less than 10%.

2. The active energy ray curable composition according to claim 1, wherein the polymerizable photoinitiator (A) and/or the polymerizable compound (B) have one or more covalent bonds of heteroatoms and hydrogen atoms per one molecule.

3. The active energy ray curable composition according to claims 1 or 2, wherein the polymerizable photoinitiator (A) and/or the polymerizable compound (B) have one or more ethylenically unsaturated groups which may be selected from (meth)acrylamide group, (meth)acrylate group, vinyl group, vinyl ether group, alkyl vinyl ether group, allyl group, (meth)allyl ether group, styryl group, and maleimide group.

4. The active energy ray curable composition according to any one of claims 1 to 3, wherein the polymerizable photoinitiator (A) and/or the polymerizable compound (B) form covalent bonds with hydrogen atoms using one or more heteroatoms which may be selected from oxygen atom, sulfur atom, nitrogen atom, phosphorus atom, boron atom, and silicon atom.

5. The active energy ray curable composition according to any one of claims 1 to 4, wherein the polymerizable photoinitiator (A) has one or more (meth)acrylamide groups as the ethylenically unsaturated groups and one or more urethane bonds and/or urea bonds as the covalent bonds between heteroatoms and hydrogen atoms.

6. An active energy ray curable ink composition containing the active energy ray curable composition according to any one of claims 1 to 5.

7. An active energy ray curable inkjet ink composition containing the active energy ray curable composition according to any one of claims 1 to 5.

8. An active energy ray curable ink composition for two dimensional or three-dimensional modeling containing the active energy ray curable composition according to any one of claims 1 to 5.

9. An active energy ray curable nail cosmetic composition containing the active energy ray curable composition according to any one of claims 1 to 5.

10. An active energy ray curable pressure-sensitive adhesive composition containing the active energy ray curable composition according to any one of claims 1 to 5.

11. An active energy ray curable adhesive composition containing the active energy ray curable composition according to any one of claims 1 to 5.

12. An active energy ray curable sealing material composition containing the active energy ray curable composition according to any one of claims 1 to 5.

13. An active energy ray curable coating agent composition containing the active energy ray curable composition according to any one of claims 1 to 5.

14. An active energy ray curable self-repairing coating material containing the active energy ray curable composition according to any one of claims 1 to 5.

15. An active energy ray curable dental composition containing the active energy ray curable composition according to any one of claims 1 to 5.

## INTERNATIONAL SEARCH REPORT

| International application No. |
| --- |
| **PCT/JP2022/039517** |

**A. CLASSIFICATION OF SUBJECT MATTER**

*C08F 2/48*(2006.01)i; *A61K 8/84*(2006.01)i; *A61Q 3/02*(2006.01)i; *B41M 5/00*(2006.01)i; *C08F 220/00*(2006.01)i; *C09D 4/00*(2006.01)i; *C09D 7/63*(2018.01)i; *C09D 11/101*(2014.01)i; *C09D 11/30*(2014.01)i; *C09D 11/38*(2014.01)i; *C09J 4/00*(2006.01)i; *C09J 7/38*(2018.01)i; *C09K 3/10*(2006.01)i
FI:  C08F2/48; A61K8/84; A61Q3/02; B41M5/00 120; C08F220/00; C09D4/00; C09D7/63; C09D11/101; C09D11/30; C09D11/38; C09J4/00; C09J7/38; C09K3/10 E

According to International Patent Classification (IPC) or to both national classification and IPC

**B. FIELDS SEARCHED**

Minimum documentation searched (classification system followed by classification symbols)

C08F2/48; A61K8/84; A61Q3/02; B41M5/00; C08F220/00; C09D4/00; C09D7/63; C09D11/101; C09D11/30; C09D11/38; C09J4/00; C09J7/38; C09K3/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2021/117880 A1 (KJ CHEMICALS CORP.) 17 June 2021 (2021-06-17) claims, paragraphs [0070], [0113], examples | 1-14 |
| Y | | 15 |
| Y | JP 2013-227368 A (SAN APRO KK) 07 November 2013 (2013-11-07) paragraph [0102] | 15 |
| A | JP 2021-95439 A (KJ CHEMICALS CORP.) 24 June 2021 (2021-06-24) entire text | 1-15 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **11 November 2022** | **22 November 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2022/039517**

| Patent document cited in search report | | | Publication date (day/month/year) | Patent family member(s) | | | Publication date (day/month/year) |
|---|---|---|---|---|---|---|---|
| WO | 2021/117880 | A1 | 17 June 2021 | TW | 202132363 | A | |
| JP | 2013-227368 | A | 07 November 2013 | (Family: none) | | | |
| JP | 2021-95439 | A | 24 June 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000159827 A
- JP 22013500303 A
- WO 2021117880 A
- JP 2010208964 A